# EUROPEAN PATENT SPECIFICATION

(11) **EP 2 463 377 B1**
(45) Date of publication and mention of the grant of the patent: **14.05.2014**
(21) Application number: 10806301.7
(22) Date of filing: 28.06.2010
(51) Int. Cl.: C12P 13/08, C12N 1/21, C12N 15/09, C12R 1/63

(54) **A method for producing L-lysine using a Vibrio bacterium**
Verfahren zur Herstellung von L-Lysin unter Verwendung eines Vibrio Bakteriums
Procédé de production de L-Lysine à l'aide d'une Vibrio bactérie

(30) Priority: 03.08.2009 JP 2009180819
(43) Date of publication of application: 13.06.2012
(73) Proprietor: Ajinomoto Co., Inc., Tokyo 104-8315 (JP)
(72) Inventor: INOUE, Ippei, Kawasaki-shi Kanagawa 210-8681 (JP); YASUEDA, Hisashi, Kawasaki-shi Kanagawa 210-8681 (JP)
(74) Representative: Strehl Schübel-Hopf & Partner
(86) International application number: PCT/JP2010/060972
(87) International publication number: WO 2011/016301

(56) References cited:
- WO-A1-2008/093829
- WO-A1-2008/093829
- LU, Z. ET AL.: 'Evolution of an Escherichia coli protein with increased resistance to oxidative stress.' J BIOL CHEM. vol. 273, 1998, pages 8308 - 8316, XP003016930
- DATABASE DATABASE DDBJ/EMBL/GENBAN [Online] 01 May 2009 MAKINO, K. ET AL.: 'Definition: alcohol dehydrogenase [Vibrio parahaemolyticus RIMD 2210633]', XP008150212 Database accession no. NP_800076

### SEQUENCE LISTING

<110> Ajinomoto Co., Inc.
<120> A method for producing L-lysine using a Vibrio bacterium
<130> EPA-65211
<150> JP2009-180819
   <151> 2009-08-03
<160> 20
<170> PatentIn version 3.5
<210> 1
   <211> 1149
   <212> DNA
   <213> Vibrio natriegens
<220>
   <221> CDS
   <222> (1)..(1149)
<400> 1
   atg act agt gca ttt ttt atc cct acg gta aac ctc atg ggc gca ggc      48
   Met Thr Ser Ala Phe Phe Ile Pro Thr Val Asn Leu Met Gly Ala Gly
   1               5                   10                  15
   tgt ctt aaa gac gcc gcg gac agt att caa tca caa ggc ttt aaa aaa      96
   Cys Leu Lys Asp Ala Ala Asp Ser Ile Gln Ser Gln Gly Phe Lys Lys
               20                  25                  30
   ggg ttg atc gtt acc gat aaa atc ctt aac caa atc ggt gtg gtt gag     144
   Gly Leu Ile Val Thr Asp Lys Ile Leu Asn Gln Ile Gly Val Val Glu
           35                  40                  45
   cag gtt caa ggc ctt ctt aac gaa aga agc gtc gca acc gta gtg ttc     192
   Gln Val Gln Gly Leu Leu Asn Glu Arg Ser Val Ala Thr Val Val Phe
       50                  55                  60
   gat ggc act cag cca aac cca acg att aac aac gtt aac gac ggc tta     240
   Asp Gly Thr Gln Pro Asn Pro Thr Ile Asn Asn Val Asn Asp Gly Leu
   65                  70                  75                  80
   gca cta tta aaa gaa aac gag tgt gac ttt gta atc tca ctt ggc ggt     288
   Ala Leu Leu Lys Glu Asn Glu Cys Asp Phe Val Ile Ser Leu Gly Gly
                   85                  90                  95
   ggc tca cca cac gac tgt gcg aaa ggt atc gca ctt gtt gct gca aac     336
   Gly Ser Pro His Asp Cys Ala Lys Gly Ile Ala Leu Val Ala Ala Asn
               100                 105                 110
   ggt gga cac att tct gac tac gag ggt gtt gat caa tct gca aaa cca     384
   Gly Gly His Ile Ser Asp Tyr Glu Gly Val Asp Gln Ser Ala Lys Pro
           115                 120                 125
   atg ctg cca cta atc gcc atc aat acg act gcg ggt act gca tcg gaa     432
   Met Leu Pro Leu Ile Ala Ile Asn Thr Thr Ala Gly Thr Ala Ser Glu
       130                 135                 140
   atg act cgt ttc tgt atc atc acc gat gaa gaa cgt cac att aag atg     480
   Met Thr Arg Phe Cys Ile Ile Thr Asp Glu Glu Arg His Ile Lys Met
   145                 150                 155                 160
   gca atc gta gat aag cac acc acg cca ttg atc tca gta aac gac gct     528
   Ala Ile Val Asp Lys His Thr Thr Pro Leu Ile Ser Val Asn Asp Ala
                   165                 170                 175
   gag tta atg ctt gca aaa cca gcg tct ctg acc gcg gca acg ggt atg     576
   Glu Leu Met Leu Ala Lys Pro Ala Ser Leu Thr Ala Ala Thr Gly Met
               180                 185                 190
   gac gcg ctg act cac gcg att gaa gcg tac gtt tct att gcg gca aca     624
   Asp Ala Leu Thr His Ala Ile Glu Ala Tyr Val Ser Ile Ala Ala Thr
           195                 200                 205
   cca att acg gat gca gtt gcg att aaa gcg att gag tta att caa gcg        672
   Pro Ile Thr Asp Ala Val Ala Ile Lys Ala Ile Glu Leu Ile Gln Ala
       210                 215                 220
   cac ctg cgc aca gca gtg aaa aac ggt gaa aat ctg gaa gct cgt gaa        720
   His Leu Arg Thr Ala Val Lys Asn Gly Glu Asn Leu Glu Ala Arg Glu
   225                 230                 235                 240
   caa atg gcg tac gcg cag ttt atg gcg ggt atg gcg ttt aac aac gct        768
   Gln Met Ala Tyr Ala Gln Phe Met Ala Gly Met Ala Phe Asn Asn Ala
                   245                 250                 255
   tca ctg ggt tac gtc cac gca atg gct cac cag tta ggt ggt ttc tac        816
   Ser Leu Gly Tyr Val His Ala Met Ala His Gln Leu Gly Gly Phe Tyr
               260                 265                 270
   gat tta cca cac ggt gtg tgt aat gcc atc ctg ctt cct cac gta caa        864
   Asp Leu Pro His Gly Val Cys Asn Ala Ile Leu Leu Pro His Val Gln
           275                 280                 285
   cgc tac aac gcg caa gtc tgc cct gaa cgt ctg act gat gtg gcg aaa        912
   Arg Tyr Asn Ala Gln Val Cys Pro Glu Arg Leu Thr Asp Val Ala Lys
       290                 295                 300
   gca atg ggc gtc aac gtg gaa ggc atg tca gca gag caa ggt gca gca        960
   Ala Met Gly Val Asn Val Glu Gly Met Ser Ala Glu Gln Gly Ala Ala
   305                 310                 315                 320
   gcc gcg atc gac gcg ata gtt aca cta gcg aaa gat gtt ggt att cct       1008
   Ala Ala Ile Asp Ala Ile Val Thr Leu Ala Lys Asp Val Gly Ile Pro
                   325                 330                 335
   gct ggc atc aag gag ctt ggc gcg aaa cta gaa gat att cca acg ctg       1056
   Ala Gly Ile Lys Glu Leu Gly Ala Lys Leu Glu Asp Ile Pro Thr Leu
               340                 345                 350
   gct gat aac gca ctt aaa gac gcg tgt ggt ttc act aac cct aaa caa       1104
   Ala Asp Asn Ala Leu Lys Asp Ala Cys Gly Phe Thr Asn Pro Lys Gln
           355                 360                 365
   gcg act cac gaa gag atc tct gca ata ttc gaa gcg gca atg taa           1149
   Ala Thr His Glu Glu Ile Ser Ala Ile Phe Glu Ala Ala Met
       370                 375                 380
<210> 2
   <211> 382
   <212> PRT
   <213> Vibrio natriegens
<400> 2
   Met Thr Ser Ala Phe Phe Ile Pro Thr Val Asn Leu Met Gly Ala Gly
   1               5                   10                  15
   Cys Leu Lys Asp Ala Ala Asp Ser Ile Gln Ser Gln Gly Phe Lys Lys
               20                  25                  30
   Gly Leu Ile Val Thr Asp Lys Ile Leu Asn Gln Ile Gly Val Val Glu
           35                  40                  45
   Gln Val Gln Gly Leu Leu Asn Glu Arg Ser Val Ala Thr Val Val Phe
       50                  55                  60
   Asp Gly Thr Gln Pro Asn Pro Thr Ile Asn Asn Val Asn Asp Gly Leu
   65                  70                  75                  80
   Ala Leu Leu Lys Glu Asn Glu Cys Asp Phe Val Ile Ser Leu Gly Gly
                   85                  90                  95
   Gly Ser Pro His Asp Cys Ala Lys Gly Ile Ala Leu Val Ala Ala Asn
               100                 105                 110
   Gly Gly His Ile Ser Asp Tyr Glu Gly Val Asp Gln Ser Ala Lys Pro
           115                 120                 125
   Met Leu Pro Leu Ile Ala Ile Asn Thr Thr Ala Gly Thr Ala Ser Glu
       130                 135                 140
   Met Thr Arg Phe Cys Ile Ile Thr Asp Glu Glu Arg His Ile Lys Met
   145                 150                 155                 160
   Ala Ile Val Asp Lys His Thr Thr Pro Leu Ile Ser Val Asn Asp Ala
                   165                 170                 175
   Glu Leu Met Leu Ala Lys Pro Ala Ser Leu Thr Ala Ala Thr Gly Met
               180                 185                 190
   Asp Ala Leu Thr His Ala Ile Glu Ala Tyr Val Ser Ile Ala Ala Thr
           195                 200                 205
   Pro Ile Thr Asp Ala Val Ala Ile Lys Ala Ile Glu Leu Ile Gln Ala
       210                 215                 220
   His Leu Arg Thr Ala Val Lys Asn Gly Glu Asn Leu Glu Ala Arg Glu
   225                 230                 235                 240
   Gln Met Ala Tyr Ala Gln Phe Met Ala Gly Met Ala Phe Asn Asn Ala
                   245                 250                 255
   Ser Leu Gly Tyr Val His Ala Met Ala His Gln Leu Gly Gly Phe Tyr
               260                 265                 270
   Asp Leu Pro His Gly Val Cys Asn Ala Ile Leu Leu Pro His Val Gln
           275                 280                 285
   Arg Tyr Asn Ala Gln Val Cys Pro Glu Arg Leu Thr Asp Val Ala Lys
       290                 295                 300
   Ala Met Gly Val Asn Val Glu Gly Met Ser Ala Glu Gln Gly Ala Ala
   305                 310                 315                 320
   Ala Ala Ile Asp Ala Ile Val Thr Leu Ala Lys Asp Val Gly Ile Pro
                   325                 330                 335
   Ala Gly Ile Lys Glu Leu Gly Ala Lys Leu Glu Asp Ile Pro Thr Leu
               340                 345                 350
   Ala Asp Asn Ala Leu Lys Asp Ala Cys Gly Phe Thr Asn Pro Lys Gln
           355                 360                 365
   Ala Thr His Glu Glu Ile Ser Ala Ile Phe Glu Ala Ala Met
       370                 375                 380
<210> 3
   <211> 28
   <212> DNA
   <213> Artificial
<220>
   <223> primer
<400> 3
   aaagaattcg acgtccacat atacctgc 28
<210> 4
   <211> 28
   <212> DNA
   <213> Artificial
<220>
   <223> primer
<400> 4
   aaagaattcg acgtcaatgc caatagga 28
<210> 5
   <211> 29
   <212> DNA
   <213> Artificial
<220>
   <223> primer
<400> 5
   aaagtcgacg aggaaatgga tgagaccag 29
<210> 6
   <211> 30
   <212> DNA
   <213> Artificial
<220>
   <223> primer
<400> 6
   tctgattcga attggattgc tccgtcgttc 30
<210> 7
   <211> 29
   <212> DNA
   <213> Artificial
<220>
   <223> primer
<400> 7
   aaagcatgct tcgagccttt ctttatcag 29
<210> 8
   <211> 30
   <212> DNA
   <213> Artificial
<220>
   <223> primer
<400> 8
   gcaatccaat tcgaatcaga gttcactctc 30
<210> 9
   <211> 17
   <212> DNA
   <213> Artificial
<220>
   <223> primer
<400> 9
   tatcgaccag ttgcatg 17
<210> 10
   <211> 17
   <212> DNA
   <213> Artificial
<220>
   <223> primer
<400> 10
   ttcgcttcgc caacatc 17
<210> 11
   <211> 1779
   <212> DNA
   <213> Vibrio natriegens
<220>
   <221> CDS
   <222> (526)..(1710)
<400> 11
   agtacgcgtt atacgactca ctatagggag atctagaagg tgctgacagc aaaaacctac      60
   gtactatggt tgatgatatc aagaaccaaa tgggttctgg tgttgtactt ctggctaaca     120
   tcacgggtga caaagttggt ttgatcgctg gtgtaactaa agatctggtt ggcaaagtca     180
   aagcgggtga tttagttaaa atggtagcag agcaggttgg tggtaaaggc ggcggtcgtc     240
   ctgatatggc gcaagctggc ggtactgacg tagcggcact tccagaagca atcaaaactg     300
   ttcagccttg gctagaagag cgcttgtaac aagctataaa taacaaatat acgctcaatc     360
   aatttgtttt gattgggcgt aattttttta agttgaccaa gtggtttaat ggaccagaga     420
   ttaagctggc ccactaagct gcttggtttt tcttgtaact accacttata taaacagaat     480
   gtatgtggtc cgaatgagac tttggtcttg ggaaggtgaa gactg gtg aaa aag ccc     537
                                                     Val Lys Lys Pro
                                                     1
   ctt atc gtg caa aag ttt ggc gga acc tct gtg ggt tca att gaa aga     585
   Leu Ile Val Gln Lys Phe Gly Gly Thr Ser Val Gly Ser Ile Glu Arg
   5                   10                  15                  20
   atc cac caa gtt gct gaa cac atc att aag gcg aaa aat gat ggt aat     633
   Ile His Gln Val Ala Glu His Ile Ile Lys Ala Lys Asn Asp Gly Asn
                   25                  30                  35
   caa gtt gtt gta gtt gtg tct gct atg tca ggc gaa acc aat agg ctt     681
   Gln Val Val Val Val Val Ser Ala Met Ser Gly Glu Thr Asn Arg Leu
               40                  45                  50
   atg gac cta gct aaa cag gta gat agc gtt cct aca gcc cga gaa ctt     729
   Met Asp Leu Ala Lys Gln Val Asp Ser Val Pro Thr Ala Arg Glu Leu
           55                  60                  65
   gat gtt ttg ctc tct gct ggt gag caa gtg tcg atg gca ctg ttg gcg     777
   Asp Val Leu Leu Ser Ala Gly Glu Gln Val Ser Met Ala Leu Leu Ala
       70                  75                  80
   atg aca ctg aac aaa atg ggt cat cct gca cgc tca ctt acc gga gca     825
   Met Thr Leu Asn Lys Met Gly His Pro Ala Arg Ser Leu Thr Gly Ala
   85                  90                  95                  100
   cag gcg aac att gtg act gat aac cag cac aat gac gca acg att aag     873
   Gln Ala Asn Ile Val Thr Asp Asn Gln His Asn Asp Ala Thr Ile Lys
                   105                 110                 115
   cac att gat act acg aga gtg atg gca ttg ctt gaa caa gag cat gtc     921
   His Ile Asp Thr Thr Arg Val Met Ala Leu Leu Glu Gln Glu His Val
               120                 125                 130
   gtt atc gtc gca ggt ttt cag ggt gtg aat gaa aat ggg gat atc acc     969
   Val Ile Val Ala Gly Phe Gln Gly Val Asn Glu Asn Gly Asp Ile Thr
           135                 140                 145
   aca tta ggt cga gga ggc tca gat acg agc gca gtg aca ctg gct ggt    1017
   Thr Leu Gly Arg Gly Gly Ser Asp Thr Ser Ala Val Thr Leu Ala Gly
       150                 155                 160
   gca cta cgt gct gat gag tgt caa atc ttt acc gac gtt gat ggt att    1065
   Ala Leu Arg Ala Asp Glu Cys Gln Ile Phe Thr Asp Val Asp Gly Ile
   165                 170                 175                 180
   tat act tgt gac cca cgt gta gta aaa acg gcg caa aaa atg gca gtg    1113
   Tyr Thr Cys Asp Pro Arg Val Val Lys Thr Ala Gln Lys Met Ala Val
                   185                 190                 195
   att gac ttt cct tca atg gaa gca atg gca agc cgt ggt gct aaa gtt    1161
   Ile Asp Phe Pro Ser Met Glu Ala Met Ala Ser Arg Gly Ala Lys Val
               200                 205                 210
   tta cat tta cct tcg gtg caa tat gca tgg aag aac aat gta cca cta    1209
   Leu His Leu Pro Ser Val Gln Tyr Ala Trp Lys Asn Asn Val Pro Leu
           215                 220                 225
   cgc gtg ctt tct aca ttc gat gtg aat gaa ggt agt tta gtt aag gga    1257
   Arg Val Leu Ser Thr Phe Asp Val Asn Glu Gly Ser Leu Val Lys Gly
       230                 235                 240
   gag att ggc aca caa gcc atc tct ggt ata gct att caa cga gac ctt    1305
   Glu Ile Gly Thr Gln Ala Ile Ser Gly Ile Ala Ile Gln Arg Asp Leu
   245                 250                 255                 260
   gcc att att gaa gta gat aaa gaa cat ttg tcc agt gct aca aag caa    1353
   Ala Ile Ile Glu Val Asp Lys Glu His Leu Ser Ser Ala Thr Lys Gln
                   265                 270                 275
   tgt cag atg cta ggt atc gat gtc tgg aat gtg atc gag gaa aca gaa    1401
   Cys Gln Met Leu Gly Ile Asp Val Trp Asn Val Ile Glu Glu Thr Glu
               280                 285                 290
   cgg aca ggt atc atg ata aaa caa gat gca tgt gcc aag ttc gat ctg     1449
   Arg Thr Gly Ile Met Ile Lys Gln Asp Ala Cys Ala Lys Phe Asp Leu
           295                 300                 305
   gtg ttc agc gat aaa atc cgt aat agt gaa atg gta agc ctg ttg acc     1497
   Val Phe Ser Asp Lys Ile Arg Asn Ser Glu Met Val Ser Leu Leu Thr
       310                 315                 320
   gca gta ggg ctt gag gcc aat gga atg gtc gag cat gca tgc gat ttg     1545
   Ala Val Gly Leu Glu Ala Asn Gly Met Val Glu His Ala Cys Asp Leu
   325                 330                 335                 340
   ctg gct gag caa gat att gcg atc aat ttc tgc gca acg aat gca ctc     1593
   Leu Ala Glu Gln Asp Ile Ala Ile Asn Phe Cys Ala Thr Asn Ala Leu
                   345                 350                 355
   act atg atg cta gta tta tct cct gac tgt gtg gat atg gcg gca aac     1641
   Thr Met Met Leu Val Leu Ser Pro Asp Cys Val Asp Met Ala Ala Asn
               360                 365                 370
   att ctt cat gat gct tac att acg tct agt gaa gcg tta agc att cag     1689
   Ile Leu His Asp Ala Tyr Ile Thr Ser Ser Glu Ala Leu Ser Ile Gln
           375                 380                 385
   caa aaa cat gcc cta ata ggg tgatttcgct aacaaggtag tttacgaaaa        1740
   Gln Lys His Ala Leu Ile Gly
       390                 395
   tataactttt gttggataat agcgtgatag caagaaaat                          1779
<210> 12
   <211> 395
   <212> PRT
   <213> Vibrio natriegens
<400> 12
   Val Lys Lys Pro Leu Ile Val Gln Lys Phe Gly Gly Thr Ser Val Gly
   1               5                   10                  15
   Ser Ile Glu Arg Ile His Gln Val Ala Glu His Ile Ile Lys Ala Lys
               20                  25                  30
   Asn Asp Gly Asn Gln Val Val Val Val Val Ser Ala Met Ser Gly Glu
           35                  40                  45
   Thr Asn Arg Leu Met Asp Leu Ala Lys Gln Val Asp Ser Val Pro Thr
       50                  55                  60
   Ala Arg Glu Leu Asp Val Leu Leu Ser Ala Gly Glu Gln Val Ser Met
   65                  70                  75                  80
   Ala Leu Leu Ala Met Thr Leu Asn Lys Met Gly His Pro Ala Arg Ser
                   85                  90                  95
   Leu Thr Gly Ala Gln Ala Asn Ile Val Thr Asp Asn Gln His Asn Asp
               100                 105                 110
   Ala Thr Ile Lys His Ile Asp Thr Thr Arg Val Met Ala Leu Leu Glu
           115                 120                 125
   Gln Glu His Val Val Ile Val Ala Gly Phe Gln Gly Val Asn Glu Asn
       130                 135                 140
   Gly Asp Ile Thr Thr Leu Gly Arg Gly Gly Ser Asp Thr Ser Ala Val
   145                 150                 155                 160
   Thr Leu Ala Gly Ala Leu Arg Ala Asp Glu Cys Gln Ile Phe Thr Asp
                   165                 170                 175
   Val Asp Gly Ile Tyr Thr Cys Asp Pro Arg Val Val Lys Thr Ala Gln
               180                 185                 190
   Lys Met Ala Val Ile Asp Phe Pro Ser Met Glu Ala Met Ala Ser Arg
           195                 200                 205
   Gly Ala Lys Val Leu His Leu Pro Ser Val Gln Tyr Ala Trp Lys Asn
       210                 215                 220
   Asn Val Pro Leu Arg Val Leu Ser Thr Phe Asp Val Asn Glu Gly Ser
   225                 230                 235                 240
   Leu Val Lys Gly Glu Ile Gly Thr Gln Ala Ile Ser Gly Ile Ala Ile
                   245                 250                 255
   Gln Arg Asp Leu Ala Ile Ile Glu Val Asp Lys Glu His Leu Ser Ser
               260                 265                 270
   Ala Thr Lys Gln Cys Gln Met Leu Gly Ile Asp Val Trp Asn Val Ile
           275                 280                 285
   Glu Glu Thr Glu Arg Thr Gly Ile Met Ile Lys Gln Asp Ala Cys Ala
       290                 295                 300
   Lys Phe Asp Leu Val Phe Ser Asp Lys Ile Arg Asn Ser Glu Met Val
   305                 310                 315                 320
   Ser Leu Leu Thr Ala Val Gly Leu Glu Ala Asn Gly Met Val Glu His
                   325                 330                 335
   Ala Cys Asp Leu Leu Ala Glu Gln Asp Ile Ala Ile Asn Phe Cys Ala
               340                 345                 350
   Thr Asn Ala Leu Thr Met Met Leu Val Leu Ser Pro Asp Cys Val Asp
           355                 360                 365
   Met Ala Ala Asn Ile Leu His Asp Ala Tyr Ile Thr Ser Ser Glu Ala
       370                 375                 380
   Leu Ser Ile Gln Gln Lys His Ala Leu Ile Gly
   385                 390                 395
<210> 13
   <211> 3547
   <212> DNA
   <213> Vibrio natriegens
<220>
   <221> CDS
   <222> (486)..(2942)
<400> 13
   ttaaattgtt gtgcaagagt ggttgctctg cattttcttt gatctttgcc gttttagcca      60
   agatcgccat gaatgtgggt ggaatgtaac tttttctatg caaagctatt tcgggaatga     120
   aatactaaga taaatttatt aaaaaaattg aattttaatt tatttctttg ttttcagtga     180
   tatatagcag aatttatctc tgcaagactg gaaaataaac aatttttttg ttgactctgt     240
   tacagaaact cggtaaacgt agggttaagc aaacacacaa ttcgtagaat attaatttat     300
   gccagttaac agcctcgttg taatgaccac caccattatt attaccgaca ttacaattgt     360
   aggggcaggc tgctgagcga aagaaattca caaaaaaggc ctgtatccaa caagatacag     420
   gccttttttt atgctttctt aatacaaaat ttagacaagt ttaagaagat acactggagg     480
   aaggg atg cga gta ttg aag ttt ggc ggt tca tca ttg gct gat gca gat      530
         Met Arg Val Leu Lys Phe Gly Gly Ser Ser Leu Ala Asp Ala Asp
         1               5                   10                  15
   cgc ttt tta aga gca gca gat atc att gcg aat aat gct caa cag gaa        578
   Arg Phe Leu Arg Ala Ala Asp Ile Ile Ala Asn Asn Ala Gln Gln Glu
                   20                  25                  30
   gaa gtc gca gtc gtg ctt tct gca ccg ggt aaa aca act aat aaa ctg        626
   Glu Val Ala Val Val Leu Ser Ala Pro Gly Lys Thr Thr Asn Lys Leu
               35                  40                  45
   gtt gct gtg att gaa ggc gct ttg cga aat ggt gaa gca gaa tta caa        674
   Val Ala Val Ile Glu Gly Ala Leu Arg Asn Gly Glu Ala Glu Leu Gln
           50                  55                  60
   att aac gag tta gaa gaa tcg ttt aaa acg ctt ttt gcc gat atc caa        722
   Ile Asn Glu Leu Glu Glu Ser Phe Lys Thr Leu Phe Ala Asp Ile Gln
       65                  70                  75
   gcc ttg gtt cct aat ctg gat ggg act ggc tac gac aat caa gtt aaa        770
   Ala Leu Val Pro Asn Leu Asp Gly Thr Gly Tyr Asp Asn Gln Val Lys
   80                  85                  90                  95
   acc tcg ctc tct caa tta cgt caa ttt gtc cat ggc atc agc ctg tta        818
   Thr Ser Leu Ser Gln Leu Arg Gln Phe Val His Gly Ile Ser Leu Leu
                   100                 105                 110
   ggc atg tgt cca aac aat gtg aat gcg cgc atc atc agt aaa ggt gaa        866
   Gly Met Cys Pro Asn Asn Val Asn Ala Arg Ile Ile Ser Lys Gly Glu
               115                 120                 125
   cgt gtt tct att caa cta atg aaa gcg gta ctt gaa gca aaa ggt caa        914
   Arg Val Ser Ile Gln Leu Met Lys Ala Val Leu Glu Ala Lys Gly Gln
           130                 135                 140
   aaa gcc agc cta att gat ccg gtt gaa tac ctt tat gct caa ggc gat        962
   Lys Ala Ser Leu Ile Asp Pro Val Glu Tyr Leu Tyr Ala Gln Gly Asp
       145                 150                 155
   cat ctt gaa gcg atg gtt gat gtt gat att tct aca caa aac ttc cgc      1010
   His Leu Glu Ala Met Val Asp Val Asp Ile Ser Thr Gln Asn Phe Arg
   160                 165                 170                 175
   caa aag cct ctt cct caa ggt cat gtc aac atc atg cct ggt ttt act      1058
   Gln Lys Pro Leu Pro Gln Gly His Val Asn Ile Met Pro Gly Phe Thr
                   180                 185                 190
   gcc ggc aat gag aaa gga gaa ctg gtt act cta ggt cgt aac ggt tca      1106
   Ala Gly Asn Glu Lys Gly Glu Leu Val Thr Leu Gly Arg Asn Gly Ser
               195                 200                 205
   gac tat tcg gct gcg gtt ttg gct gct tgt ctt cgc gcc gat tgt tgt      1154
   Asp Tyr Ser Ala Ala Val Leu Ala Ala Cys Leu Arg Ala Asp Cys Cys
           210                 215                 220
   gaa atc tgg act gac gtt gat ggc gta tac aac tgt gat cct cgc ttg      1202
   Glu Ile Trp Thr Asp Val Asp Gly Val Tyr Asn Cys Asp Pro Arg Leu
       225                 230                 235
   gta gaa gat gca cga cta tta aaa tca ctg agc tat cag gaa gcg atg      1250
   Val Glu Asp Ala Arg Leu Leu Lys Ser Leu Ser Tyr Gln Glu Ala Met
   240                 245                 250                 255
   gag ttg tct tac ttc ggt gcg tcc gtt ctg cac ccg aaa acc atc gct      1298
   Glu Leu Ser Tyr Phe Gly Ala Ser Val Leu His Pro Lys Thr Ile Ala
                   260                 265                 270
   cca att gct caa ttc cac att cca tgc tta atc aag aac agt ttt aac      1346
   Pro Ile Ala Gln Phe His Ile Pro Cys Leu Ile Lys Asn Ser Phe Asn
               275                 280                 285
   ccg caa ggt gca ggt acg ctg att ggc caa gat act ggt gaa gat aac      1394
   Pro Gln Gly Ala Gly Thr Leu Ile Gly Gln Asp Thr Gly Glu Asp Asn
           290                 295                 300
   ctg gct atc aag ggt att act act ctg aat gac ctg acc atg gtg aac      1442
   Leu Ala Ile Lys Gly Ile Thr Thr Leu Asn Asp Leu Thr Met Val Asn
       305                 310                 315
   gtt tct ggg cca ggt atg aaa ggc atg gtt ggc atg gcg agc cgt gta      1490
   Val Ser Gly Pro Gly Met Lys Gly Met Val Gly Met Ala Ser Arg Val
   320                 325                 330                 335
   ttt ggt gct atg tct tca gcg ggt gtt tca atc gta ctg atc act cag      1538
   Phe Gly Ala Met Ser Ser Ala Gly Val Ser Ile Val Leu Ile Thr Gln
                   340                 345                 350
   tca tca tca gaa tac agc atc agc ttc tgt atc gaa gca gaa gat aaa      1586
   Ser Ser Ser Glu Tyr Ser Ile Ser Phe Cys Ile Glu Ala Glu Asp Lys
               355                 360                 365
   gca aaa gcg cag cag aca ctg gca gaa gcg ttt gag ctt gaa ctg aaa      1634
   Ala Lys Ala Gln Gln Thr Leu Ala Glu Ala Phe Glu Leu Glu Leu Lys
           370                 375                 380
   gat ggt ctg ctt gaa cca gta gag ttt atc gat gat gtt gct atc gtg      1682
   Asp Gly Leu Leu Glu Pro Val Glu Phe Ile Asp Asp Val Ala Ile Val
       385                 390                 395
   acc ctt gtt ggt gac ggc atg cgt aca tca cgt ggt gtg gct tca cgt      1730
   Thr Leu Val Gly Asp Gly Met Arg Thr Ser Arg Gly Val Ala Ser Arg
   400                 405                 410                 415
   ttc ttc tca tct ctt gct gaa gta aac gtc aac att gtt gca atc gca      1778
   Phe Phe Ser Ser Leu Ala Glu Val Asn Val Asn Ile Val Ala Ile Ala
                   420                 425                 430
   cag ggt tct tca gag cgc gct atc tca gcg gta atc cca gaa gat aag      1826
   Gln Gly Ser Ser Glu Arg Ala Ile Ser Ala Val Ile Pro Glu Asp Lys
               435                 440                 445
   atc tct gaa gca atc aaa gcc tgt cac atg aac ctg ttc aat tct aaa      1874
   Ile Ser Glu Ala Ile Lys Ala Cys His Met Asn Leu Phe Asn Ser Lys
           450                 455                 460
   cac ttc ctt gat gtt ttc gtc gta ggc atc ggc ggt gtt ggt ggc gag      1922
   His Phe Leu Asp Val Phe Val Val Gly Ile Gly Gly Val Gly Gly Glu
       465                 470                 475
   ctg gtg gat cag att gaa cgc caa caa gct aaa ctg gca gaa aaa ggc      1970
   Leu Val Asp Gln Ile Glu Arg Gln Gln Ala Lys Leu Ala Glu Lys Gly
   480                 485                 490                 495
   atc atc att cgc gta tgt ggt ctg gca aac agt aag ggt ctg ctt ctg      2018
   Ile Ile Ile Arg Val Cys Gly Leu Ala Asn Ser Lys Gly Leu Leu Leu
                   500                 505                 510
   gat agt gaa ggg ctg cca ctg gaa cac tgg cgc gac cgc atg tct tca      2066
   Asp Ser Glu Gly Leu Pro Leu Glu His Trp Arg Asp Arg Met Ser Ser
               515                 520                 525
   gcc act gaa gag ttc agc cta gcg cgc ctg att gca ctt gtg caa cgt      2114
   Ala Thr Glu Glu Phe Ser Leu Ala Arg Leu Ile Ala Leu Val Gln Arg
           530                 535                 540
   aac cac atc att aac cct gtt ttg gtt gat tgt act tcg agt gaa gcc      2162
   Asn His Ile Ile Asn Pro Val Leu Val Asp Cys Thr Ser Ser Glu Ala
       545                 550                 555
   att gct aac cag tac gct gat ttc ttg gcg gca ggt ttc cac gtt gtt      2210
   Ile Ala Asn Gln Tyr Ala Asp Phe Leu Ala Ala Gly Phe His Val Val
   560                 565                 570                 575
   act cca aat aag aaa gcc aat aca gca agc atg gct tac tac cac cag      2258
   Thr Pro Asn Lys Lys Ala Asn Thr Ala Ser Met Ala Tyr Tyr His Gln
                   580                 585                 590
   ctt cgc gat gta gca cgc agc tct cgt cgt aaa ttg atg tac gag aca      2306
   Leu Arg Asp Val Ala Arg Ser Ser Arg Arg Lys Leu Met Tyr Glu Thr
               595                 600                 605
   acg gtt ggt gcg ggt ttg ccg gtt atc gaa aac ctg cag aat ctc att      2354
   Thr Val Gly Ala Gly Leu Pro Val Ile Glu Asn Leu Gln Asn Leu Ile
           610                 615                 620
   tca gcg ggt gat gag ctt gaa cga ttc acc ggc att ttg tct ggt tca      2402
   Ser Ala Gly Asp Glu Leu Glu Arg Phe Thr Gly Ile Leu Ser Gly Ser
       625                 630                 635
   cta tct tac atc ttc ggt aag cta gac gaa ggc atg agc ttg agc gaa      2450
   Leu Ser Tyr Ile Phe Gly Lys Leu Asp Glu Gly Met Ser Leu Ser Glu
   640                 645                 650                 655
   gcg acc aac atc gcg aaa gaa aac ggc ttc acc gaa ccg gat cct cgt      2498
   Ala Thr Asn Ile Ala Lys Glu Asn Gly Phe Thr Glu Pro Asp Pro Arg
                   660                 665                 670
   gat gat cta tcg ggt atg gat gtg gct cgt aag ctt ctt att ctg gcg      2546
   Asp Asp Leu Ser Gly Met Asp Val Ala Arg Lys Leu Leu Ile Leu Ala
               675                 680                 685
   cgt gaa gcg gga atg tcg cta gag ttg gaa gac gtt gtg gtt gat caa      2594
   Arg Glu Ala Gly Met Ser Leu Glu Leu Glu Asp Val Val Val Asp Gln
           690                 695                 700
   gcg ctg cca ccg ggc ttt gat gat tca ggc agt gtt gat gag ttt atg      2642
   Ala Leu Pro Pro Gly Phe Asp Asp Ser Gly Ser Val Asp Glu Phe Met
       705                 710                 715
   gcg aga ctt cct gaa gct gat gcg tac ttt aaa gat ctt gtc gca aaa      2690
   Ala Arg Leu Pro Glu Ala Asp Ala Tyr Phe Lys Asp Leu Val Ala Lys
   720                 725                 730                 735
   gcc gct gaa gag ggt aaa gtt tta cgt tat gtt ggt gaa atc aat gat      2738
   Ala Ala Glu Glu Gly Lys Val Leu Arg Tyr Val Gly Glu Ile Asn Asp
                   740                 745                 750
   ggt aag tgc aaa gtc agc att gct atc gtt gat gaa aat gat cca atg      2786
   Gly Lys Cys Lys Val Ser Ile Ala Ile Val Asp Glu Asn Asp Pro Met
               755                 760                 765
   ttc aaa att aaa gat ggt gag aac gct ctg gcg ttt tac agc cgt tac      2834
   Phe Lys Ile Lys Asp Gly Glu Asn Ala Leu Ala Phe Tyr Ser Arg Tyr
           770                 775                 780
   tac caa cca atc cca ttg gta ctt cgt ggt tac ggt gca ggt acg gaa      2882
   Tyr Gln Pro Ile Pro Leu Val Leu Arg Gly Tyr Gly Ala Gly Thr Glu
       785                 790                 795
   gtt acc gca gca ggc gta ttt tca gat gtg atg cgt act tta ggt tgg      2930
   Val Thr Ala Ala Gly Val Phe Ser Asp Val Met Arg Thr Leu Gly Trp
   800                 805                 810                 815
   aaa cta ggg gta taacagtaat gagttcaagt gatatggatg tagtggttta          2982
   Lys Leu Gly Val
   tgccccagca tcaattggta atgtaagcgt aggttttgat gttttgggag cggctgtctc   3042
   tcctatcgat ggcaccttat tgggtgatcg agtcctagtg aaatctggtt ctgagccatt   3102
   tagcttgaaa accgcaggta acttcgtctc gaagttacct acagaaccaa aagaaaacat   3162
   cgtttacgac tgttgggttg tatttgctcg cgagctagat agaaagggcg cagagctgaa   3222
   accattggaa atgacactcg agaagaacat gcctatcggt tctggtcttg gttccagtgc   3282
   ttgttctatt gttgctgcat tagatgcttt gaaccgtttt cacgggcaac cacttaatga   3342
   aactgagctg ttagcgctaa tgggcgagat ggaaggtaaa atttctggtg gtatccatta   3402
   tgacaacgtt gcaccttgtt acttaggtgg cgtgcagttg atgcttgaag agcttggtat   3462
   cattagccag gaagtgccat gttttgatga ctggtactgg gtaatggctt atccgggaat   3522
   caaagtgtct actgctgaag ctcga                                          3547
<210> 14
   <211> 819
   <212> PRT
   <213> Vibrio natriegens
<400> 14
   Met Arg Val Leu Lys Phe Gly Gly Ser Ser Leu Ala Asp Ala Asp Arg
   1               5                   10                  15
   Phe Leu Arg Ala Ala Asp Ile Ile Ala Asn Asn Ala Gln Gln Glu Glu
               20                  25                  30
   Val Ala Val Val Leu Ser Ala Pro Gly Lys Thr Thr Asn Lys Leu Val
           35                  40                  45
   Ala Val Ile Glu Gly Ala Leu Arg Asn Gly Glu Ala Glu Leu Gln Ile
       50                  55                  60
   Asn Glu Leu Glu Glu Ser Phe Lys Thr Leu Phe Ala Asp Ile Gln Ala
   65                  70                  75                  80
   Leu Val Pro Asn Leu Asp Gly Thr Gly Tyr Asp Asn Gln Val Lys Thr
                   85                  90                  95
   Ser Leu Ser Gln Leu Arg Gln Phe Val His Gly Ile Ser Leu Leu Gly
               100                 105                 110
   Met Cys Pro Asn Asn Val Asn Ala Arg Ile Ile Ser Lys Gly Glu Arg
           115                 120                 125
   Val Ser Ile Gln Leu Met Lys Ala Val Leu Glu Ala Lys Gly Gln Lys
       130                 135                 140
   Ala Ser Leu Ile Asp Pro Val Glu Tyr Leu Tyr Ala Gln Gly Asp His
   145                 150                 155                 160
   Leu Glu Ala Met Val Asp Val Asp Ile Ser Thr Gln Asn Phe Arg Gln
                   165                 170                 175
   Lys Pro Leu Pro Gln Gly His Val Asn Ile Met Pro Gly Phe Thr Ala
               180                 185                 190
   Gly Asn Glu Lys Gly Glu Leu Val Thr Leu Gly Arg Asn Gly Ser Asp
           195                 200                 205
   Tyr Ser Ala Ala Val Leu Ala Ala Cys Leu Arg Ala Asp Cys Cys Glu
       210                 215                 220
   Ile Trp Thr Asp Val Asp Gly Val Tyr Asn Cys Asp Pro Arg Leu Val
   225                 230                 235                 240
   Glu Asp Ala Arg Leu Leu Lys Ser Leu Ser Tyr Gln Glu Ala Met Glu
                   245                 250                 255
   Leu Ser Tyr Phe Gly Ala Ser Val Leu His Pro Lys Thr Ile Ala Pro
               260                 265                 270
   Ile Ala Gln Phe His Ile Pro Cys Leu Ile Lys Asn Ser Phe Asn Pro
           275                 280                 285
   Gln Gly Ala Gly Thr Leu Ile Gly Gln Asp Thr Gly Glu Asp Asn Leu
       290                 295                 300
   Ala Ile Lys Gly Ile Thr Thr Leu Asn Asp Leu Thr Met Val Asn Val
   305                 310                 315                 320
   Ser Gly Pro Gly Met Lys Gly Met Val Gly Met Ala Ser Arg Val Phe
                   325                 330                 335
   Gly Ala Met Ser Ser Ala Gly Val Ser Ile Val Leu Ile Thr Gln Ser
               340                 345                 350
   Ser Ser Glu Tyr Ser Ile Ser Phe Cys Ile Glu Ala Glu Asp Lys Ala
           355                 360                 365
   Lys Ala Gln Gln Thr Leu Ala Glu Ala Phe Glu Leu Glu Leu Lys Asp
       370                 375                 380
   Gly Leu Leu Glu Pro Val Glu Phe Ile Asp Asp Val Ala Ile Val Thr
   385                 390                 395                 400
   Leu Val Gly Asp Gly Met Arg Thr Ser Arg Gly Val Ala Ser Arg Phe
                   405                 410                 415
   Phe Ser Ser Leu Ala Glu Val Asn Val Asn Ile Val Ala Ile Ala Gln
               420                 425                 430
   Gly Ser Ser Glu Arg Ala Ile Ser Ala Val Ile Pro Glu Asp Lys Ile
           435                 440                 445
   Ser Glu Ala Ile Lys Ala Cys His Met Asn Leu Phe Asn Ser Lys His
       450                 455                 460
   Phe Leu Asp Val Phe Val Val Gly Ile Gly Gly Val Gly Gly Glu Leu
   465                 470                 475                 480
   Val Asp Gln Ile Glu Arg Gln Gln Ala Lys Leu Ala Glu Lys Gly Ile
                   485                 490                 495
   Ile Ile Arg Val Cys Gly Leu Ala Asn Ser Lys Gly Leu Leu Leu Asp
               500                 505                 510
   Ser Glu Gly Leu Pro Leu Glu His Trp Arg Asp Arg Met Ser Ser Ala
           515                 520                 525
   Thr Glu Glu Phe Ser Leu Ala Arg Leu Ile Ala Leu Val Gln Arg Asn
       530                 535                 540
   His Ile Ile Asn Pro Val Leu Val Asp Cys Thr Ser Ser Glu Ala Ile
   545                 550                 555                 560
   Ala Asn Gln Tyr Ala Asp Phe Leu Ala Ala Gly Phe His Val Val Thr
                   565                 570                 575
   Pro Asn Lys Lys Ala Asn Thr Ala Ser Met Ala Tyr Tyr His Gln Leu
               580                 585                 590
   Arg Asp Val Ala Arg Ser Ser Arg Arg Lys Leu Met Tyr Glu Thr Thr
           595                 600                 605
   Val Gly Ala Gly Leu Pro Val Ile Glu Asn Leu Gln Asn Leu Ile Ser
       610                 615                 620
   Ala Gly Asp Glu Leu Glu Arg Phe Thr Gly Ile Leu Ser Gly Ser Leu
   625                 630                 635                 640
   Ser Tyr Ile Phe Gly Lys Leu Asp Glu Gly Met Ser Leu Ser Glu Ala
                   645                 650                 655
   Thr Asn Ile Ala Lys Glu Asn Gly Phe Thr Glu Pro Asp Pro Arg Asp
               660                 665                 670
   Asp Leu Ser Gly Met Asp Val Ala Arg Lys Leu Leu Ile Leu Ala Arg
           675                 680                 685
   Glu Ala Gly Met Ser Leu Glu Leu Glu Asp Val Val Val Asp Gln Ala
       690                 695                 700
   Leu Pro Pro Gly Phe Asp Asp Ser Gly Ser Val Asp Glu Phe Met Ala
   705                 710                 715                 720
   Arg Leu Pro Glu Ala Asp Ala Tyr Phe Lys Asp Leu Val Ala Lys Ala
                   725                 730                 735
   Ala Glu Glu Gly Lys Val Leu Arg Tyr Val Gly Glu Ile Asn Asp Gly
               740                 745                 750
   Lys Cys Lys Val Ser Ile Ala Ile Val Asp Glu Asn Asp Pro Met Phe
           755                 760                 765
   Lys Ile Lys Asp Gly Glu Asn Ala Leu Ala Phe Tyr Ser Arg Tyr Tyr
       770                 775                 780
   Gln Pro Ile Pro Leu Val Leu Arg Gly Tyr Gly Ala Gly Thr Glu Val
   785                 790                 795                 800
   Thr Ala Ala Gly Val Phe Ser Asp Val Met Arg Thr Leu Gly Trp Lys
                   805                 810                 815
   Leu Gly Val
<210> 15
   <211> 3214
   <212> DNA
   <213> Vibrio natriegens
<220>
   <221> CDS
   <222> (376)..(2781)
<400> 15
   ctgcagaagc aatctttggt ggcgaaaatt tatcatccaa gtcttccaca gcaccctggc     60
   catgagattg ctaaaaagca acagtctggt tttggctcta tgttgagctt tgagtttgcg    120
   ggctcgtttg agcagcttaa agcctttgtc aaagagcttc agttgttctc acttgcagag    180
   tctctgggcg gggtagaaag cttgatttgt cacccagcat ctatgacgca tcgcgcgatg    240
   ggtgaagaag ccttggctga agcgggtgta tcacagcaat tgctgcgcct ttctgtaggg    300
   ctagaagatg cacaagatct tattgccgac ctagaacaag cattcactaa atctgcacag    360
   caataggagt taata atg act gta caa cgt cag cta cac aag ttt ggt ggt     411
                    Met Thr Val Gln Arg Gln Leu His Lys Phe Gly Gly
                    1               5                   10
   agc agc ctg gca aac cca gag tgt tac tta cgt gta gcg gga att ctc      459
   Ser Ser Leu Ala Asn Pro Glu Cys Tyr Leu Arg Val Ala Gly Ile Leu
           15                  20                  25
   aag gaa tac tct gca gaa aat gac ttg gtg gtg gtc tct gca gca ggt      507
   Lys Glu Tyr Ser Ala Glu Asn Asp Leu Val Val Val Ser Ala Ala Gly
       30                  35                  40
   aaa aca acc aac cgc ctg att gag ttc ctt gaa ggc tta gaa aaa gat      555
   Lys Thr Thr Asn Arg Leu Ile Glu Phe Leu Glu Gly Leu Glu Lys Asp
   45                  50                  55                  60
   ggt cga att gcg cac gaa gct ttg cag agc ttg aga cag ttt caa atc      603
   Gly Arg Ile Ala His Glu Ala Leu Gln Ser Leu Arg Gln Phe Gln Ile
                   65                  70                  75
   agt ctc att gaa gag tta tta gag gga gac gcg caa gaa cag ctg ctt      651
   Ser Leu Ile Glu Glu Leu Leu Glu Gly Asp Ala Gln Glu Gln Leu Leu
               80                  85                  90
   gcc tcc tta caa gat gaa ttc agt aca tta gcg gaa ctg acc tct ccg      699
   Ala Ser Leu Gln Asp Glu Phe Ser Thr Leu Ala Glu Leu Thr Ser Pro
           95                  100                 105
   ctg acc gaa gcg caa aag gcg gcg gta tta ggc cac gga gaa gta tgg      747
   Leu Thr Glu Ala Gln Lys Ala Ala Val Leu Gly His Gly Glu Val Trp
       110                 115                 120
   tct tct cga tta ctg gcc gct tta ctg acg caa aag caa ctg cct gct      795
   Ser Ser Arg Leu Leu Ala Ala Leu Leu Thr Gln Lys Gln Leu Pro Ala
   125                 130                 135                 140
   gta gcg caa gat gcg cga gca ttc cta cgc gca gaa gcg ggc act caa      843
   Val Ala Gln Asp Ala Arg Ala Phe Leu Arg Ala Glu Ala Gly Thr Gln
                   145                 150                 155
   cca gaa gtt gat cgt gct cgt tca tat ccg ctt atc aaa gag gca ctg      891
   Pro Glu Val Asp Arg Ala Arg Ser Tyr Pro Leu Ile Lys Glu Ala Leu
               160                 165                 170
   gca caa cat agc cac aaa cgt gtg atc att acc ggc ttc atg gca caa      939
   Ala Gln His Ser His Lys Arg Val Ile Ile Thr Gly Phe Met Ala Gln
           175                 180                 185
   aac gat aac ggt gaa act gtt ctg ctt ggc cgc aac ggt tca gac tac      987
   Asn Asp Asn Gly Glu Thr Val Leu Leu Gly Arg Asn Gly Ser Asp Tyr
       190                 195                 200
   tct gca acc gta att ggt gcg ctt gca gaa gtg agc aca gtg acg att     1035
   Ser Ala Thr Val Ile Gly Ala Leu Ala Glu Val Ser Thr Val Thr Ile
   205                 210                 215                 220
   tgg agt gat gtg gct ggt gtt tat agc gca gac cct cgt ttg gta tca     1083
   Trp Ser Asp Val Ala Gly Val Tyr Ser Ala Asp Pro Arg Leu Val Ser
                   225                 230                 235
   gat gca tgt tta cta cct ctc tta agg ctg gat gaa gcg agt gag ctg     1131
   Asp Ala Cys Leu Leu Pro Leu Leu Arg Leu Asp Glu Ala Ser Glu Leu
               240                 245                 250
   gct cgt tta gct gcg cca gtt ctg cac agc cga acc ttg cag cct gtg     1179
   Ala Arg Leu Ala Ala Pro Val Leu His Ser Arg Thr Leu Gln Pro Val
           255                 260                 265
   gct caa agc acc atg gat ctg agc ctt aaa tgc agt tac ctg cca gag     1227
   Ala Gln Ser Thr Met Asp Leu Ser Leu Lys Cys Ser Tyr Leu Pro Glu
       270                 275                 280
   tca ggt tcg acc cgc att gag cga gtt ctg gct tct ggt cgc ggg gcg     1275
   Ser Gly Ser Thr Arg Ile Glu Arg Val Leu Ala Ser Gly Arg Gly Ala
   285                 290                 295                 300
   aaa atc atc acc tca ctg gat gac gtg cta ttg gtt cag cta gca ttt     1323
   Lys Ile Ile Thr Ser Leu Asp Asp Val Leu Leu Val Gln Leu Ala Phe
                   305                 310                 315
   gcg cat gga cat gac ttt gag aaa gca cag agt gat gtt ctg gaa tcc      1371
   Ala His Gly His Asp Phe Glu Lys Ala Gln Ser Asp Val Leu Glu Ser
               320                 325                 330
   cta aaa cgc gtt cag ttg gag cca ctg gca ttt gaa gct cag cct gaa      1419
   Leu Lys Arg Val Gln Leu Glu Pro Leu Ala Phe Glu Ala Gln Pro Glu
           335                 340                 345
   cag cag ata tta cga ctg gca tac aca gca gaa att gct ggc ggt gca      1467
   Gln Gln Ile Leu Arg Leu Ala Tyr Thr Ala Glu Ile Ala Gly Gly Ala
       350                 355                 360
   ctc aag tat cta cag gaa tct gat ctg gat gcg gag att aac ctc act      1515
   Leu Lys Tyr Leu Gln Glu Ser Asp Leu Asp Ala Glu Ile Asn Leu Thr
   365                 370                 375                 380
   gat ggt tac tct ctg att gct gcc gta ggt gct ggt gtg aca act aat      1563
   Asp Gly Tyr Ser Leu Ile Ala Ala Val Gly Ala Gly Val Thr Thr Asn
                   385                 390                 395
   gcc aac cac tgt ttt ggt ttt cag caa aag ctg aaa cac tct ccg gtg      1611
   Ala Asn His Cys Phe Gly Phe Gln Gln Lys Leu Lys His Ser Pro Val
               400                 405                 410
   gaa ttt atc gct gag acg gag tct ggt tta agt ctg gtc gca gta tta      1659
   Glu Phe Ile Ala Glu Thr Glu Ser Gly Leu Ser Leu Val Ala Val Leu
           415                 420                 425
   cgt aac act gat acc gaa gaa ctg gta cag acg gtt cac agc cag tta      1707
   Arg Asn Thr Asp Thr Glu Glu Leu Val Gln Thr Val His Ser Gln Leu
       430                 435                 440
   ttt cag gca cag aag cgc gtt gct gtc gca tta tgc ggg aaa ggg aat      1755
   Phe Gln Ala Gln Lys Arg Val Ala Val Ala Leu Cys Gly Lys Gly Asn
   445                 450                 455                 460
   atc ggt tcg agc tgg ctg agt tta ttc gct tca cag aag aat gaa ctt      1803
   Ile Gly Ser Ser Trp Leu Ser Leu Phe Ala Ser Gln Lys Asn Glu Leu
                   465                 470                 475
   gaa aaa cgt cat ggc atg agc ttt gac tta gtc gcg gtg gtt gat agc      1851
   Glu Lys Arg His Gly Met Ser Phe Asp Leu Val Ala Val Val Asp Ser
               480                 485                 490
   caa atg tat tgg ttt gat agc caa ggc att gat gca tcc agt gtt tca      1899
   Gln Met Tyr Trp Phe Asp Ser Gln Gly Ile Asp Ala Ser Ser Val Ser
           495                 500                 505
   gca cgc ttc aat gac gaa agt atc gca aac gat ggc tct tgg tta tct      1947
   Ala Arg Phe Asn Asp Glu Ser Ile Ala Asn Asp Gly Ser Trp Leu Ser
       510                 515                 520
   cgt ctt ggt gct ttg cag gac tac gat gaa gct gtg gtc ctc gat gtt      1995
   Arg Leu Gly Ala Leu Gln Asp Tyr Asp Glu Ala Val Val Leu Asp Val
   525                 530                 535                 540
   acc gcc agt cag gaa tta gcg aaa tgt tac gtt gat atc gct cag caa      2043
   Thr Ala Ser Gln Glu Leu Ala Lys Cys Tyr Val Asp Ile Ala Gln Gln
                   545                 550                 555
   ggt att cac cta att tca gcg aat aaa gtc gca ggt tca gct gat agt      2091
   Gly Ile His Leu Ile Ser Ala Asn Lys Val Ala Gly Ser Ala Asp Ser
               560                 565                 570
   cag tat tac cat cag gtc cag gat gcg ttt gcc aaa att gga cgt cac      2139
   Gln Tyr Tyr His Gln Val Gln Asp Ala Phe Ala Lys Ile Gly Arg His
           575                 580                 585
   tgg ctg tat aac gct acg gta ggt gcc ggg tta cca atc aac cat acg      2187
   Trp Leu Tyr Asn Ala Thr Val Gly Ala Gly Leu Pro Ile Asn His Thr
       590                 595                 600
   gtg cgc gat ctt cgt gag agt ggt gat gag att att gca ctc tct ggt      2235
   Val Arg Asp Leu Arg Glu Ser Gly Asp Glu Ile Ile Ala Leu Ser Gly
   605                 610                 615                 620
   att ttc tcc ggt acg ctt tca tgg tta ttc caa cag ttt gat ggc tca      2283
   Ile Phe Ser Gly Thr Leu Ser Trp Leu Phe Gln Gln Phe Asp Gly Ser
                   625                 630                 635
   gtg cca ttc agc gag tta gtg gat tta gcc tgg cag caa gga tta aca      2331
   Val Pro Phe Ser Glu Leu Val Asp Leu Ala Trp Gln Gln Gly Leu Thr
               640                 645                 650
   gaa cct gac cct cgc tct gac tta gat ggc tct gat gtg atg cgt aag     2379
   Glu Pro Asp Pro Arg Ser Asp Leu Asp Gly Ser Asp Val Met Arg Lys
           655                 660                     665
   ctg gtt att ctg gcg cgt gag tct ggt ctg gac atc gag ccg gga agc     2427
   Leu Val Ile Leu Ala Arg Glu Ser Gly Leu Asp Ile Glu Pro Gly Ser
       670                 675                 680
   gta aag gta gag tcg tta gtt cct gaa gaa ctg cgc acg ctc agc tta     2475
   Val Lys Val Glu Ser Leu Val Pro Glu Glu Leu Arg Thr Leu Ser Leu
   685                 690                 695                 700
   gat gag ttt ttt gat aac gcc gca ctg ctg agc cag act ctg caa gaa     2523
   Asp Glu Phe Phe Asp Asn Ala Ala Leu Leu Ser Gln Thr Leu Gln Glu
                   705                 710                 715
   cgt ttg tcc aaa gcg cag aaa aac gat cag gta ctg cgt tac gtt gcg     2571
   Arg Leu Ser Lys Ala Gln Lys Asn Asp Gln Val Leu Arg Tyr Val Ala
               720                 725                 730
   cgt tta gag aaa gat ggc aaa gcg acc gtc ggc att gaa gcc ttg tcc     2619
   Arg Leu Glu Lys Asp Gly Lys Ala Thr Val Gly Ile Glu Ala Leu Ser
           735                 740                 745
   cgt gag cat gcg ctg gct aac cta ctg ccg tgt gac aat att ttt gct     2667
   Arg Glu His Ala Leu Ala Asn Leu Leu Pro Cys Asp Asn Ile Phe Ala
       750                 755                 760
   att gag agc aag tgg tac aaa gac aac ccg ctt gtt atc cgc ggt cct     2715
   Ile Glu Ser Lys Trp Tyr Lys Asp Asn Pro Leu Val Ile Arg Gly Pro
   765                 770                 775                 780
   ggc gct gga cgt gaa gtt acg gca ggg gcg att cag tcc gat tta aac     2763
   Gly Ala Gly Arg Glu Val Thr Ala Gly Ala Ile Gln Ser Asp Leu Asn
                   785                 790                 795
   cgc ctt gct ggg cta ttc taaacactca aaaccccgga attcatccgg            2811
   Arg Leu Ala Gly Leu Phe
               800
   ggttttttat tcctgtcttg gtgccataat taatctcaga accgttattg gcgaactatt    2871
   tttgtggata aatattagcc aacaatcaac ttgaactatc ctcatcatta acgtgaaaaa   2931
   aattcataat cacaaggttg acattaaatc gtattcaata cattctgtag acatatagac   2991
   gtctaaacgt cgatttgaga atttgatttc gtggcgggtt gccacaggga gagtaagatg   3051
   ggttacacgc acgcaggcca tatcgatgcc ttaaaccaga atatcgctga attgtcagac   3111
   aatattaatg tttcatttga gttttttccg ccaagcaatg aaaagatgga agagacgcta    3171
   tggaactctg tgcaccgtct aaaaacactc aagcctaagt ttg                     3214
<210> 16
   <211> 802
   <212> PRT
   <213> Vibrio natriegens
<400> 16
   Met Thr Val Gln Arg Gln Leu His Lys Phe Gly Gly Ser Ser Leu Ala
   1               5                   10                  15
   Asn Pro Glu Cys Tyr Leu Arg Val Ala Gly Ile Leu Lys Glu Tyr Ser
               20                  25                  30
   Ala Glu Asn Asp Leu Val Val Val Ser Ala Ala Gly Lys Thr Thr Asn
           35                  40                  45
   Arg Leu Ile Glu Phe Leu Glu Gly Leu Glu Lys Asp Gly Arg Ile Ala
       50                  55                  60
   His Glu Ala Leu Gln Ser Leu Arg Gln Phe Gln Ile Ser Leu Ile Glu
   65                  70                  75                  80
   Glu Leu Leu Glu Gly Asp Ala Gln Glu Gln Leu Leu Ala Ser Leu Gln
                   85                  90                  95
   Asp Glu Phe Ser Thr Leu Ala Glu Leu Thr Ser Pro Leu Thr Glu Ala
               100                 105                 110
   Gln Lys Ala Ala Val Leu Gly His Gly Glu Val Trp Ser Ser Arg Leu
           115                 120                 125
   Leu Ala Ala Leu Leu Thr Gln Lys Gln Leu Pro Ala Val Ala Gln Asp
       130                 135                 140
   Ala Arg Ala Phe Leu Arg Ala Glu Ala Gly Thr Gln Pro Glu Val Asp
   145                 150                 155                 160
   Arg Ala Arg Ser Tyr Pro Leu Ile Lys Glu Ala Leu Ala Gln His Ser
                   165                 170                 175
   His Lys Arg Val Ile Ile Thr Gly Phe Met Ala Gln Asn Asp Asn Gly
               180                 185                 190
   Glu Thr Val Leu Leu Gly Arg Asn Gly Ser Asp Tyr Ser Ala Thr Val
           195                 200                 205
   Ile Gly Ala Leu Ala Glu Val Ser Thr Val Thr Ile Trp Ser Asp Val
       210                 215                 220
   Ala Gly Val Tyr Ser Ala Asp Pro Arg Leu Val Ser Asp Ala Cys Leu
   225                 230                 235                 240
   Leu Pro Leu Leu Arg Leu Asp Glu Ala Ser Glu Leu Ala Arg Leu Ala
                   245                 250                 255
   Ala Pro Val Leu His Ser Arg Thr Leu Gln Pro Val Ala Gln Ser Thr
               260                 265                 270
   Met Asp Leu Ser Leu Lys Cys Ser Tyr Leu Pro Glu Ser Gly Ser Thr
           275                 280                 285
   Arg Ile Glu Arg Val Leu Ala Ser Gly Arg Gly Ala Lys Ile Ile Thr
       290                 295                 300
   Ser Leu Asp Asp Val Leu Leu Val Gln Leu Ala Phe Ala His Gly His
   305                 310                 315                 320
   Asp Phe Glu Lys Ala Gln Ser Asp Val Leu Glu Ser Leu Lys Arg Val
                   325                 330                 335
   Gln Leu Glu Pro Leu Ala Phe Glu Ala Gln Pro Glu Gln Gln Ile Leu
               340                 345                 350
   Arg Leu Ala Tyr Thr Ala Glu Ile Ala Gly Gly Ala Leu Lys Tyr Leu
           355                 360                 365
   Gln Glu Ser Asp Leu Asp Ala Glu Ile Asn Leu Thr Asp Gly Tyr Ser
       370                 375                 380
   Leu Ile Ala Ala Val Gly Ala Gly Val Thr Thr Asn Ala Asn His Cys
   385                 390                 395                 400
   Phe Gly Phe Gln Gln Lys Leu Lys His Ser Pro Val Glu Phe Ile Ala
                   405                 410                 415
   Glu Thr Glu Ser Gly Leu Ser Leu Val Ala Val Leu Arg Asn Thr Asp
               420                 425                 430
   Thr Glu Glu Leu Val Gln Thr Val His Ser Gln Leu Phe Gln Ala Gln
           435                 440                 445
   Lys Arg Val Ala Val Ala Leu Cys Gly Lys Gly Asn Ile Gly Ser Ser
       450                 455                 460
   Trp Leu Ser Leu Phe Ala Ser Gln Lys Asn Glu Leu Glu Lys Arg His
   465                 470                 475                 480
   Gly Met Ser Phe Asp Leu Val Ala Val Val Asp Ser Gln Met Tyr Trp
                   485                 490                 495
   Phe Asp Ser Gln Gly Ile Asp Ala Ser Ser Val Ser Ala Arg Phe Asn
               500                 505                 510
   Asp Glu Ser Ile Ala Asn Asp Gly Ser Trp Leu Ser Arg Leu Gly Ala
           515                 520                 525
   Leu Gln Asp Tyr Asp Glu Ala Val Val Leu Asp Val Thr Ala Ser Gln
       530                 535                 540
   Glu Leu Ala Lys Cys Tyr Val Asp Ile Ala Gln Gln Gly Ile His Leu
   545                 550                 555                 560
   Ile Ser Ala Asn Lys Val Ala Gly Ser Ala Asp Ser Gln Tyr Tyr His
                   565                 570                 575
   Gln Val Gln Asp Ala Phe Ala Lys Ile Gly Arg His Trp Leu Tyr Asn
               580                 585                 590
   Ala Thr Val Gly Ala Gly Leu Pro Ile Asn His Thr Val Arg Asp Leu
           595                 600                 605
   Arg Glu Ser Gly Asp Glu Ile Ile Ala Leu Ser Gly Ile Phe Ser Gly
       610                 615                 620
   Thr Leu Ser Trp Leu Phe Gln Gln Phe Asp Gly Ser Val Pro Phe Ser
   625                 630                 635                 640
   Glu Leu Val Asp Leu Ala Trp Gln Gln Gly Leu Thr Glu Pro Asp Pro
                   645                 650                 655
   Arg Ser Asp Leu Asp Gly Ser Asp Val Met Arg Lys Leu Val Ile Leu
               660                 665                 670
   Ala Arg Glu Ser Gly Leu Asp Ile Glu Pro Gly Ser Val Lys Val Glu
           675                     680                     685
   Ser Leu Val Pro Glu Glu Leu Arg Thr Leu Ser Leu Asp Glu Phe Phe
       690                     695                     700
   Asp Asn Ala Ala Leu Leu Ser Gln Thr Leu Gln Glu Arg Leu Ser Lys
   705                     710                     715                     720
   Ala Gln Lys Asn Asp Gln Val Leu Arg Tyr Val Ala Arg Leu Glu Lys
                   725                     730                     735
   Asp Gly Lys Ala Thr Val Gly Ile Glu Ala Leu Ser Arg Glu His Ala
               740                     745                     750
   Leu Ala Asn Leu Leu Pro Cys Asp Asn Ile Phe Ala Ile Glu Ser Lys
           755                     760                     765
   Trp Tyr Lys Asp Asn Pro Leu Val Ile Arg Gly Pro Gly Ala Gly Arg
       770                     775                     780
   Glu Val Thr Ala Gly Ala Ile Gln Ser Asp Leu Asn Arg Leu Ala Gly
   785                     790                     795                     800
   Leu Phe
<210> 17
   <211> 2648
   <212> DNA
   <213> Vibrio natriegens
<220>
   <221> misc_feature
   <222> (862)
   <223> n = a, t, g or c
<220>
   <221> CDS
   <222> (1060)..(2409)
<400> 17
   ataactacgg ccagtgccaa gcttgcatgc ctgcaggtcg actctagagg atccccttgt      60
   gttgcttggc taacttaccc aaagcttgag cgtcgctgac cgcacctgtc gatgtttccg     120
   cgtgaacaaa tgctaaaatt tttgtgtcag gatgctgctg cagtgcttgt tcgactttat     180
   cgacagagac tggcgcgccc cactcgtcat caaccaagac aacttcacca ccagcacgaa     240
   caacgttttc acgcatgcgc tcaccaaaaa caccatttcg gcatacgata actttgtcac     300
   ctttctcgat aaggttaaca aaacatgcct ccatacccgc actacctgga gcagaaacgg     360
   caatagtaaa gtcgttttct gtctggaacg cgtactttag caattgtttc agctcatcca     420
   tcattgcgat aaacagaggg tccaagtgac caaccgttgg gcggctaagc gcttgcagca     480
   cttgcggata gatatctgaa ggacctggtc ccattagggt acggtgaggg ggaataaagc     540
   tttggatcgg catgtgagct ccttgtttga cgttacttaa cccgagcgcg gacaatagtt     600
   tttagaattt tgaataagtc tcgggcatca aagaactcac actaatggat tttatagtgc     660
   tcggcaatta gccgtaggta ttgagttcaa accactgtca caatttcact aataaaaaat     720
   cttggaatag ttgaaatatt cattgacttt gtgacgccaa aaccgttcaa tgtttgagct     780
   ttatgtggaa gaggagcact acccaggtag ataatttgtg gaaccgcaat tccaatacga     840
   attattcagg gggagtagtg cncgaggtaa gtcaaatttg cagggtttga cttgtcggtt     900
   gacgtgggtt gagtcccatc aactgtcatc agcattgtct gatgaagagc ttctgagggt     960
   acatatttca aatatcactc taaatacctc tctttttgct ctgttccttt cctcactaaa    1020
   tatcggatat attagtttaa gaagtcgtgg gagaactca gtg agc gca ttt aac       1074
                                               Val Ser Ala Phe Asn
                                               1               5
   gta gca aaa ttc ggt gga acc agt gtt gcc aat ttc gag gca atg agc      1122
   Val Ala Lys Phe Gly Gly Thr Ser Val Ala Asn Phe Glu Ala Met Ser
                   10                  15                  20
   cgt tgt gcc gcc att att gaa aac aat cca aat act cgc ctt gtc gtt      1170
   Arg Cys Ala Ala Ile Ile Glu Asn Asn Pro Asn Thr Arg Leu Val Val
               25                  30                  35
   agc agc gct tgc tct ggt gta acg aac cta ttg gta gaa ctg gcc aat      1218
   Ser Ser Ala Cys Ser Gly Val Thr Asn Leu Leu Val Glu Leu Ala Asn
           40                  45                  50
   ggt gtt cag gat cag gaa cag cgt gcc gag ttg cta cgt aag cta gca      1266
   Gly Val Gln Asp Gln Glu Gln Arg Ala Glu Leu Leu Arg Lys Leu Ala
       55                  60                  65
   gaa att cat gat gac atc ctt agt caa cta aga gat gca gca gaa gca      1314
   Glu Ile His Asp Asp Ile Leu Ser Gln Leu Arg Asp Ala Ala Glu Ala
   70                  75                  80                  85
   agt gca gag gtt tac gca atc ctt gat acg gta acc agc ctt gca gaa      1362
   Ser Ala Glu Val Tyr Ala Ile Leu Asp Thr Val Thr Ser Leu Ala Glu
                   90                  95                  100
   gca gct tct atc caa gct agc tct aaa ctg aca gac cat tta gtt gcg      1410
   Ala Ala Ser Ile Gln Ala Ser Ser Lys Leu Thr Asp His Leu Val Ala
               105                 110                 115
   tgt ggt gag tta atg tcg acg cac att ttg gct caa ctg atg aga gag      1458
   Cys Gly Glu Leu Met Ser Thr His Ile Leu Ala Gln Leu Met Arg Glu
           120                 125                 130
   cgt ggt atc aat gcg gtg cgt ttt gat att cgt gat gtg cta aga acc      1506
   Arg Gly Ile Asn Ala Val Arg Phe Asp Ile Arg Asp Val Leu Arg Thr
       135                 140                 145
   gac gat aac ttc gga cgt gct gag cca aat gtc gaa gcg att tct cag      1554
   Asp Asp Asn Phe Gly Arg Ala Glu Pro Asn Val Glu Ala Ile Ser Gln
   150                 155                 160                 165
   cta gct caa gag aag tta gtc cct cta tgt cag gag tca gta gtc att      1602
   Leu Ala Gln Glu Lys Leu Val Pro Leu Cys Gln Glu Ser Val Val Ile
                   170                 175                 180
   act caa ggc ttc atc ggc tca gac gaa gaa ggc aat aca acc aca tta      1650
   Thr Gln Gly Phe Ile Gly Ser Asp Glu Glu Gly Asn Thr Thr Thr Leu
               185                 190                 195
   ggt cgt ggt ggc agt gac tac agt gcc gca ctc att gct gaa ggc gtt      1698
   Gly Arg Gly Gly Ser Asp Tyr Ser Ala Ala Leu Ile Ala Glu Gly Val
           200                 205                 210
   aaa gct tct ggc tta gaa atc tgg act gat gtt ccg ggc atc tac aca      1746
   Lys Ala Ser Gly Leu Glu Ile Trp Thr Asp Val Pro Gly Ile Tyr Thr
       215                 220                 225
   aca gac cca cgt att gcg cca aaa gcg tct cct att cca gag atc agc      1794
   Thr Asp Pro Arg Ile Ala Pro Lys Ala Ser Pro Ile Pro Glu Ile Ser
   230                 235                 240                 245
   ttt agc gaa gcg tca gaa atg gca aac ttc ggt gct aag atc ctc cac      1842
   Phe Ser Glu Ala Ser Glu Met Ala Asn Phe Gly Ala Lys Ile Leu His
                   250                 255                 260
   cct tct aca ctc gtt cct gct ttg cgc cac gac att cca gtg ttt gtt      1890
   Pro Ser Thr Leu Val Pro Ala Leu Arg His Asp Ile Pro Val Phe Val
               265                 270                 275
   ggc tct tct aaa gat cca gaa gca ggt ggc aca tgg att cgt cac caa      1938
   Gly Ser Ser Lys Asp Pro Glu Ala Gly Gly Thr Trp Ile Arg His Gln
           280                 285                 290
   gta gaa agc tcg ccg cta tac cga gct ctt gca ttg cgt tgc aac caa      1986
   Val Glu Ser Ser Pro Leu Tyr Arg Ala Leu Ala Leu Arg Cys Asn Gln
       295                 300                 305
   acc atg gtc act cta cgt agt gca agc atg ttc cat gca tac ggc ttc      2034
   Thr Met Val Thr Leu Arg Ser Ala Ser Met Phe His Ala Tyr Gly Phe
   310                 315                 320                 325
   ctg gct aaa gtg ttc gag att ctg gct aaa cac aaa att tca gtg gac      2082
   Leu Ala Lys Val Phe Glu Ile Leu Ala Lys His Lys Ile Ser Val Asp
                   330                 335                 340
   ctg att acc act tca gaa atc agt gtt tcg tta act cta gac caa aca      2130
   Leu Ile Thr Thr Ser Glu Ile Ser Val Ser Leu Thr Leu Asp Gln Thr
               345                 350                 355
   gac acc tct ggt ggt gca cca caa cta cct caa gcc gta aga gaa gag      2178
   Asp Thr Ser Gly Gly Ala Pro Gln Leu Pro Gln Ala Val Arg Glu Glu
           360                 365                 370
   cta gaa gaa ttg tgt aaa gtg gaa gta gag cac gac cta tgc ttg gtg      2226
   Leu Glu Glu Leu Cys Lys Val Glu Val Glu His Asp Leu Cys Leu Val
       375                 380                 385
   gct ctt atc gga aac aac atg agc ggt agc aaa gga tac gcc aaa caa      2274
   Ala Leu Ile Gly Asn Asn Met Ser Gly Ser Lys Gly Tyr Ala Lys Gln
   390                 395                 400                 405
   gta ttc ggt aca tta gaa gac ttt aac ctg cgt atg att tgc tac ggt      2322
   Val Phe Gly Thr Leu Glu Asp Phe Asn Leu Arg Met Ile Cys Tyr Gly
                   410                 415                 420
   gcg agc ccg cat aac ttg tgc ttc cta ctg cac aag tca gaa tcg caa      2370
   Ala Ser Pro His Asn Leu Cys Phe Leu Leu His Lys Ser Glu Ser Gln
               425                 430                 435
   cag gcg att caa aaa ctg cat gct gag cta ttt gaa aaa taaacgctgt       2419
   Gln Ala Ile Gln Lys Leu His Ala Glu Leu Phe Glu Lys
           440                 445                 450
   tcatctcaca atataaaaag ggctgcctga tggcagccct ttctctttcc gttggattag   2479
   ccgttaatat tcggacctaa ccatttttct gcttcaagcc tgtcccaacc tttacgttca   2539
   gcatagcttt ctaactgatc ttcctggatc tgcgcgatcg caaagtagcg agaatccgga   2599
   tgagagaagt accaaccaga cactgaggca ccagggtaca tggcataga                2648
<210> 18
   <211> 450
   <212> PRT
   <213> Vibrio natriegens
<400> 18
   Val Ser Ala Phe Asn Val Ala Lys Phe Gly Gly Thr Ser Val Ala Asn
   1               5                   10                  15
   Phe Glu Ala Met Ser Arg Cys Ala Ala Ile Ile Glu Asn Asn Pro Asn
               20                  25                  30
   Thr Arg Leu Val Val Ser Ser Ala Cys Ser Gly Val Thr Asn Leu Leu
           35                  40                  45
   Val Glu Leu Ala Asn Gly Val Gln Asp Gln Glu Gln Arg Ala Glu Leu
       50                  55                  60
   Leu Arg Lys Leu Ala Glu Ile His Asp Asp Ile Leu Ser Gln Leu Arg
   65                  70                  75                  80
   Asp Ala Ala Glu Ala Ser Ala Glu Val Tyr Ala Ile Leu Asp Thr Val
                   85                  90                  95
   Thr Ser Leu Ala Glu Ala Ala Ser Ile Gln Ala Ser Ser Lys Leu Thr
               100                 105                 110
   Asp His Leu Val Ala Cys Gly Glu Leu Met Ser Thr His Ile Leu Ala
           115                 120                 125
   Gln Leu Met Arg Glu Arg Gly Ile Asn Ala Val Arg Phe Asp Ile Arg
       130                 135                 140
   Asp Val Leu Arg Thr Asp Asp Asn Phe Gly Arg Ala Glu Pro Asn Val
   145                 150                 155                 160
   Glu Ala Ile Ser Gln Leu Ala Gln Glu Lys Leu Val Pro Leu Cys Gln
                   165                 170                 175
   Glu Ser Val Val Ile Thr Gln Gly Phe Ile Gly Ser Asp Glu Glu Gly
               180                 185                 190
   Asn Thr Thr Thr Leu Gly Arg Gly Gly Ser Asp Tyr Ser Ala Ala Leu
           195                 200                 205
   Ile Ala Glu Gly Val Lys Ala Ser Gly Leu Glu Ile Trp Thr Asp Val
       210                 215                 220
   Pro Gly Ile Tyr Thr Thr Asp Pro Arg Ile Ala Pro Lys Ala Ser Pro
   225                 230                 235                 240
   Ile Pro Glu Ile Ser Phe Ser Glu Ala Ser Glu Met Ala Asn Phe Gly
                   245                 250                 255
   Ala Lys Ile Leu His Pro Ser Thr Leu Val Pro Ala Leu Arg His Asp
               260                 265                 270
   Ile Pro Val Phe Val Gly Ser Ser Lys Asp Pro Glu Ala Gly Gly Thr
           275                 280                 285
   Trp Ile Arg His Gln Val Glu Ser Ser Pro Leu Tyr Arg Ala Leu Ala
       290                 295                 300
   Leu Arg Cys Asn Gln Thr Met Val Thr Leu Arg Ser Ala Ser Met Phe
   305                 310                 315                 320
   His Ala Tyr Gly Phe Leu Ala Lys Val Phe Glu Ile Leu Ala Lys His
                   325                 330                 335
   Lys Ile Ser Val Asp Leu Ile Thr Thr Ser Glu Ile Ser Val Ser Leu
               340                 345                 350
   Thr Leu Asp Gln Thr Asp Thr Ser Gly Gly Ala Pro Gln Leu Pro Gln
           355                 360                 365
   Ala Val Arg Glu Glu Leu Glu Glu Leu Cys Lys Val Glu Val Glu His
       370                 375                 380
   Asp Leu Cys Leu Val Ala Leu Ile Gly Asn Asn Met Ser Gly Ser Lys
   385                 390                 395                 400
   Gly Tyr Ala Lys Gln Val Phe Gly Thr Leu Glu Asp Phe Asn Leu Arg
                   405                 410                 415
   Met Ile Cys Tyr Gly Ala Ser Pro His Asn Leu Cys Phe Leu Leu His
               420                 425                 430
   Lys Ser Glu Ser Gln Gln Ala Ile Gln Lys Leu His Ala Glu Leu Phe
           435                 440                 445
   Glu Lys
       450
<210> 19
   <211> 2331
   <212> DNA
   <213> Vibrio natriegens
<220>
   <221> CDS
   <222> (344)..(1765)
<400> 19
   acggaacacg cacatccact acaaaaacca cttagagtca gttttctgta tgagtggtga      60
   aggcgagatt gaagtggtag gtggtgaaac ctatccaatc aaaccaggta cactgtacat     120
   tctggataaa aacgacgagc actacctaag agcatataaa aacaaagaaa tggtaatggc     180
   gtgcgtattc aacccaccta tcacaggtgc ggaagtacac gatgagaacg gcgtgtaccc     240
   tcttgttgac taaaactaaa tagtcactgt gaggtgggct tccacctcac atttcaattc     300
   tctcttcttt attttttaag ttaccaatca aaaaaggctt aac atg act ttt acc       355
                                                   Met Thr Phe Thr
                                                   1
   gta gaa aaa atc ggc ggt act tca atg aca gca ttt gat gct gtt cta       403
   Val Glu Lys Ile Gly Gly Thr Ser Met Thr Ala Phe Asp Ala Val Leu
   5                   10                  15                  20
   gac aat att att ctt cgt ccc aag aca cca tac aac cga gta ttt gtt       451
   Asp Asn Ile Ile Leu Arg Pro Lys Thr Pro Tyr Asn Arg Val Phe Val
                   25                  30                  35
   gta tcg gcg tac ggt ggt atg act gac gcg cta tta gaa tgt aaa aaa       499
   Val Ser Ala Tyr Gly Gly Met Thr Asp Ala Leu Leu Glu Cys Lys Lys
               40                  45                  50
   acc agt aaa gcg ggc gta tac caa ctg gtt gct aag cgt gat gac tcg       547
   Thr Ser Lys Ala Gly Val Tyr Gln Leu Val Ala Lys Arg Asp Asp Ser
           55                  60                  65
   tgg gaa gaa gcg ttg gca tac gta gag aat cgt atg ttg ctg aca aac       595
   Trp Glu Glu Ala Leu Ala Tyr Val Glu Asn Arg Met Leu Leu Thr Asn
       70                  75                  80
   gag aat att ttc gcc gat cca atg aat cga atg cga gcg gat aag ttt       643
   Glu Asn Ile Phe Ala Asp Pro Met Asn Arg Met Arg Ala Asp Lys Phe
   85                  90                  95                  100
   att cgt tca cgc att tca gaa gcg aaa aac tgt atc gct aat att tta       691
   Ile Arg Ser Arg Ile Ser Glu Ala Lys Asn Cys Ile Ala Asn Ile Leu
                   105                 110                 115
   gaa acg tgc cag tac ggc cag ttt tca ctg cgt cac tac ttg cct caa       739
   Glu Thr Cys Gln Tyr Gly Gln Phe Ser Leu Arg His Tyr Leu Pro Gln
               120                 125                 130
   atc cgt gag ttt ctt tct tca att ggt gaa gcg cac agt gct tat aac       787
   Ile Arg Glu Phe Leu Ser Ser Ile Gly Glu Ala His Ser Ala Tyr Asn
           135                 140                 145
   acc gcg ttg aaa ctg aaa aac atg ggc att aac gct aag ttt gtc gac       835
   Thr Ala Leu Lys Leu Lys Asn Met Gly Ile Asn Ala Lys Phe Val Asp
       150                 155                 160
   ttg tca ggt tgg gat aca acc gag ccg aag agt ctt gat gaa tca atc       883
   Leu Ser Gly Trp Asp Thr Thr Glu Pro Lys Ser Leu Asp Glu Ser Ile
   165                 170                 175                 180
   agt gaa gct ttt gct gat atc gat gta tca aaa gaa cta cct atc gtg      931
   Ser Glu Ala Phe Ala Asp Ile Asp Val Ser Lys Glu Leu Pro Ile Val
                   185                 190                 195
   acg ggt tat gca tat tgt aaa gaa ggt ctt atg cat acg tat gac cga      979
   Thr Gly Tyr Ala Tyr Cys Lys Glu Gly Leu Met His Thr Tyr Asp Arg
               200                 205                 210
   ggc tac agt gag atg act ttc agc cga gtt gct tcg atc acg aaa gcg     1027
   Gly Tyr Ser Glu Met Thr Phe Ser Arg Val Ala Ser Ile Thr Lys Ala
           215                 220                 225
   aat tta gcg ata atc cac aag gaa tat cac tta agt tct gct gac cca     1075
   Asn Leu Ala Ile Ile His Lys Glu Tyr His Leu Ser Ser Ala Asp Pro
       230                 235                 240
   cgt gtt gtt ggt cct gag aaa gta ttg cca att ggc agc acg aac tac     1123
   Arg Val Val Gly Pro Glu Lys Val Leu Pro Ile Gly Ser Thr Asn Tyr
   245                 250                 255                 260
   gat gtg gct gac cag ctg gct aac ctg gga atg gaa gct att cac cct     1171
   Asp Val Ala Asp Gln Leu Ala Asn Leu Gly Met Glu Ala Ile His Pro
                   265                 270                 275
   aac gca gca gcg ggt ttg cgc gaa agt ggt atc gaa ctg cag att aaa     1219
   Asn Ala Ala Ala Gly Leu Arg Glu Ser Gly Ile Glu Leu Gln Ile Lys
               280                 285                 290
   aac aca ttt gaa cct gaa cat gaa ggt act ttg att tct tct ggt tac     1267
   Asn Thr Phe Glu Pro Glu His Glu Gly Thr Leu Ile Ser Ser Gly Tyr
           295                 300                 305
   cgt cca gag gaa gac aaa gtg gag atc atc gcg ggt aag cag aaa gtg     1315
   Arg Pro Glu Glu Asp Lys Val Glu Ile Ile Ala Gly Lys Gln Lys Val
       310                 315                 320
   ttt gcg ttg cat ctt ttt gac caa gcg atg gtt ggt aaa gta gat aac     1363
   Phe Ala Leu His Leu Phe Asp Gln Ala Met Val Gly Lys Val Asp Asn
   325                 330                 335                 340
   gtg agt tac gag ctg atg gaa atc atc tca gat gca cac gtg aca ttg     1411
   Val Ser Tyr Glu Leu Met Glu Ile Ile Ser Asp Ala His Val Thr Leu
                   345                 350                 355
   gtt ggt aaa gaa atg aac gcc aac tcg att act tac tac ctg ggc ggt     1459
   Val Gly Lys Glu Met Asn Ala Asn Ser Ile Thr Tyr Tyr Leu Gly Gly
               360                 365                 370
   aat gca gac agc tta aac aaa gtt ctg tat aaa gcg gaa aaa tgt tac     1507
   Asn Ala Asp Ser Leu Asn Lys Val Leu Tyr Lys Ala Glu Lys Cys Tyr
           375                 380                 385
   ccg aaa gca tca att aaa ggc cgt atg gta gcg ttg atc tca gcg att     1555
   Pro Lys Ala Ser Ile Lys Gly Arg Met Val Ala Leu Ile Ser Ala Ile
       390                 395                 400
   ggt tct cag att gac acc aac aaa acc ttg gcg aaa ggt gta ttg gca     1603
   Gly Ser Gln Ile Asp Thr Asn Lys Thr Leu Ala Lys Gly Val Leu Ala
   405                 410                 415                 420
   ctg atg aat agt ggt gtg acg cca gtg gct ctg cac tca tca tta cga     1651
   Leu Met Asn Ser Gly Val Thr Pro Val Ala Leu His Ser Ser Leu Arg
                   425                 430                 435
   aat gtt aat gta caa ttc gtt gtg ggt gat aaa gag tat cag cga gca     1699
   Asn Val Asn Val Gln Phe Val Val Gly Asp Lys Glu Tyr Gln Arg Ala
               440                 445                 450
   att tgt gca ctg cac gat gag ttc ttc gaa ccg gtt gag aat gct gag     1747
   Ile Cys Ala Leu His Asp Glu Phe Phe Glu Pro Val Glu Asn Ala Glu
           455                 460                 465
   tcg ata gaa gac gtt gcg taagtcaatt acacaacaag acgtgataca            1795
   Ser Ile Glu Asp Val Ala
       470
   aagggagcct ttctaggctc ctttgttgtt tgtggcttta ctgtgtttat ttcggcaatg   1855
   aaaagcggct tatttggacg ataacacacg ggctgtgtgc gtatgcttat ccgttcttta   1915
   gcttacactt tctgtcacga caggatttga gtggcgtctg gctatccaat acgcgatacc   1975
   gccccagaac acggttagca gccaaagggc agtccagtgt ttagccactt gttgccatgt   2035
   tgcgcccatt tgatttaagg cgagaaagcc cttaatcgcc caagtactcg ggcttaaatc   2095
   agcaacccaa agcaggggag ccggaatgga ttctactggc caaatgaaac cggcaaggaa   2155
   gataagtggc attgagctga ccaatacgac taaggtgacc agttctctgc gtggtaaaag   2215
   gtaaccgagc caaaaaccca aaccacagca gctcaacaga aacggaacca gcaaggaaag 2275
   cagttcagtt gcctgagcga tgtgattacg ctacctttca aagctggcac caaaat 2331
<210> 20
   <211> 474
   <212> PRT
   <213> Vibrio natriegens
<400> 20
   Met Thr Phe Thr Val Glu Lys Ile Gly Gly Thr Ser Met Thr Ala Phe
   1               5                   10                  15
   Asp Ala Val Leu Asp Asn Ile Ile Leu Arg Pro Lys Thr Pro Tyr Asn
               20                  25                  30
   Arg Val Phe Val Val Ser Ala Tyr Gly Gly Met Thr Asp Ala Leu Leu
           35                  40                  45
   Glu Cys Lys Lys Thr Ser Lys Ala Gly Val Tyr Gln Leu Val Ala Lys
       50                  55                  60
   Arg Asp Asp Ser Trp Glu Glu Ala Leu Ala Tyr Val Glu Asn Arg Met
   65                  70                  75                  80
   Leu Leu Thr Asn Glu Asn Ile Phe Ala Asp Pro Met Asn Arg Met Arg
                   85                  90                  95
   Ala Asp Lys Phe Ile Arg Ser Arg Ile Ser Glu Ala Lys Asn Cys Ile
               100                 105                 110
   Ala Asn Ile Leu Glu Thr Cys Gln Tyr Gly Gln Phe Ser Leu Arg His
           115                 120                 125
   Tyr Leu Pro Gln Ile Arg Glu Phe Leu Ser Ser Ile Gly Glu Ala His
       130                 135                 140
   Ser Ala Tyr Asn Thr Ala Leu Lys Leu Lys Asn Met Gly Ile Asn Ala
   145                 150                 155                 160
   Lys Phe Val Asp Leu Ser Gly Trp Asp Thr Thr Glu Pro Lys Ser Leu
                   165                 170                 175
   Asp Glu Ser Ile Ser Glu Ala Phe Ala Asp Ile Asp Val Ser Lys Glu
               180                 185                 190
   Leu Pro Ile Val Thr Gly Tyr Ala Tyr Cys Lys Glu Gly Leu Met His
           195                 200                 205
   Thr Tyr Asp Arg Gly Tyr Ser Glu Met Thr Phe Ser Arg Val Ala Ser
       210                 215                 220
   Ile Thr Lys Ala Asn Leu Ala Ile Ile His Lys Glu Tyr His Leu Ser
   225                 230                 235                 240
   Ser Ala Asp Pro Arg Val Val Gly Pro Glu Lys Val Leu Pro Ile Gly
                   245                 250                 255
   Ser Thr Asn Tyr Asp Val Ala Asp Gln Leu Ala Asn Leu Gly Met Glu
               260                 265                 270
   Ala Ile His Pro Asn Ala Ala Ala Gly Leu Arg Glu Ser Gly Ile Glu
           275                 280                 285
   Leu Gln Ile Lys Asn Thr Phe Glu Pro Glu His Glu Gly Thr Leu Ile
       290                 295                 300
   Ser Ser Gly Tyr Arg Pro Glu Glu Asp Lys Val Glu Ile Ile Ala Gly
   305                 310                 315                 320
   Lys Gln Lys Val Phe Ala Leu His Leu Phe Asp Gln Ala Met Val Gly
                   325                 330                 335
   Lys Val Asp Asn Val Ser Tyr Glu Leu Met Glu Ile Ile Ser Asp Ala
               340                 345                 350
   His Val Thr Leu Val Gly Lys Glu Met Asn Ala Asn Ser Ile Thr Tyr
           355                 360                 365
   Tyr Leu Gly Gly Asn Ala Asp Ser Leu Asn Lys Val Leu Tyr Lys Ala
       370                 375                 380
   Glu Lys Cys Tyr Pro Lys Ala Ser Ile Lys Gly Arg Met Val Ala Leu
   385                 390                 395                 400
   Ile Ser Ala Ile Gly Ser Gln Ile Asp Thr Asn Lys Thr Leu Ala Lys
                   405                 410                 415
   Gly Val Leu Ala Leu Met Asn Ser Gly Val Thr Pro Val Ala Leu His
               420                 425                 430
   Ser Ser Leu Arg Asn Val Asn Val Gln Phe Val Val Gly Asp Lys Glu
           435                 440                 445
   Tyr Gln Arg Ala Ile Cys Ala Leu His Asp Glu Phe Phe Glu Pro Val
       450                 455                 460
   Glu Asn Ala Glu Ser Ile Glu Asp Val Ala
   465                 470

## Description

### Technical Field

The present invention relates to a method for producing L-lysine using a *Vibrio* bacterium. L-Lysine is an industrially useful L-amino acid as an additive for animal feed, ingredient of health food, amino acid infusion, and so forth.

### Background Art

L-lysine is produced by fermentation using a coryneform bacterium belonging to the genus *Brevibacterium, Corynebacterium,* or the like, a bacterium belonging to the genus *Bacillus, Escherichia, Streptomyces, Methylobacillus,* or the like, or a filamentous fungus belonging to the genus *Penicillium,* or the like.

As methods for producing L-lysine by fermentation using a microorganism, there are methods using an auxotroph derived from a wild-type strain, a metabolic regulation mutant strain derived from a wild-type strain as a strain resistant to any of various drugs, a strain having properties of both auxotroph and metabolic regulation mutants, and so forth. Furthermore, microorganisms that can efficiently produce L-lysine have been bred using recombinant DNA techniques (Patent documents 1 to 4).

Although productivity of L-lysine has been considerably increased by such breeding of microorganisms and improvement of production method as described above, development of still more inexpensive and efficient L-lysine production methods has been desired in order to respond to further increase of the demand in future.

L-Amino acid production methods based on direct fermentation have a problem that the action of a microorganism is reduced by increase of osmotic pressure due to accumulation of L-amino acid in the medium, and thus the productivity cannot be maintained for a long period of time. As a method for solving this problem, a method for producing an L-amino acid such as L-lysine by direct fermentation using a *Vibrio* bacterium has been disclosed (Patent document 5).

As a method for producing L-lysine in which decomposition of L-lysine accumulated in the medium can be reduced, methods using a microorganism of which L-lysine decomposition ability is reduced, in particular, a microorganism belonging to the genus *Escherichia* in which the *cadA* gene or the *ldcC* gene is deleted, or expression amount or enzymatic activity of the product thereof is decreased have been known (Non-patent document 1, Patent document 4).

It is known that a *cadA* homologue gene is present in *Vibrio cholerae, Vibrio parahaemolyticus* and *Vibrio vuinificus* among the *Vibrio* bacteria (Non-patent documents 2 to 4). However, it has not been known whether L-lysine decomposition ability is attenuated or deleted by disruption of such a *cadA* homologue gene in those *Vibrio* bacteria. Moreover, presence of an L-lysine decomposition pathway other than the L-lysine decomposition pathway involving a *cadA* homologue gene product has not been known in *Vibrio* bacteria.

### Prior Art References

### Patent documents

Patent document 1: European Patent Laid-open No. 0857784
Patent document 2: Japanese Patent Laid-open (KOKAI) No. 11-192088
Patent document 3: International Patent Publication WO00/53726
Patent document 4: International Patent Publication WO96/17930
Patent document 5: International Patent Publication WO2008/093829

### Non-patent documents

Non-patent document 1: S. Meng, and G.N. Bennett, J. Bacteriol., 174, 2659-2669 (1992)
Non-patent document 2: D.S. Merrell, and A. Camilli, Mol. Microbiol., 34, 836-849 (1999)
Non-patent document 3: Y. Tanaka, et al., J. Appl. Microbiol., 104, 1283-1293 (2007)
Non-patent document 4: J.E. Rhee, et al., FEMS Microbiol. Lett., 208, 245-251 (2002)

### Summary of the Invention

### Object to be Achieved by the Invention

An object of the present invention is to provide a method for efficiently producing L-lysine using a *Vibrio* bacterium. In particular, the inventors of the present invention have already found a phenomenon that L-lysine accumulated in the medium decreases with progress of culture in the case of *Vibrio* bacteria, and one of objects of the present invention is to reduce this decrease of L-lysine.

### Means for Achieving the Object

The inventors of the present invention conducted various researches in order to achieve the aforementioned object. As a result, they specified a gene coding for a factor involved in L-lysine decomposition in *Vibrio* bacteria. And they found that, by disrupting that gene, decomposition of accumulated L-lysine can be suppressed, and L-lysine can be efficiently produced, and accomplished the present invention.

The present invention thus relates to the followings.
(1) A method for producing L-lysine, which comprises culturing a *Vibrio* bacterium having an ability to produce L-lysine in a medium to produce and accumulate L-lysine in the medium or cells of the bacterium, and collecting L-lysine from the medium or cells, wherein the *Vibrio* bacterium has been modified so that an activity of a protein encoded by the *fucO* gene is reduced.
(2) The method as mentioned above, wherein the activity of the protein is reduced by introducing a mutation into a coding region of the *fucO* gene and/or an expression control region of the gene.
(3) The method as mentioned above, wherein the *fucO* gene on a chromosome is disrupted.
(4) The method as mentioned above, wherein the protein is a protein defined in the following (A) or (B):
   (A) a protein having the amino acid sequence shown in SEQ ID NO: 2,
   (B) a protein having the amino acid sequence shown in SEQ ID NO: 2 including substitutions, deletions, insertions or additions of one or several amino acid residues, wherein the reduction of the activity in the bacterium improves the ability to produce L-lysine.
(5) The method as mentioned above, wherein the *fucO* gene is a DNA defined in the following (a) or (b):
   (a) a DNA comprising the nucleotide sequence of SEQ ID NO: 1,
   (b) a DNA which hybridizes with a DNA complementary to the nucleotide sequence of SEQ ID NO: 1
      under stringent conditions of 1 x SSC, 0.1% SDS at 60°C, and codes for a protein, wherein the reduction of the activity in the bacterium improves the ability to produce L-lysine.
(6) The method as mentioned above, wherein an activity or activities of one or two or more kinds of enzymes selected from the group consisting of dihydrodipicolinate synthase, aspartokinase, dihydrodipicolinate reductase, and diaminopimelate dehydrogenase are enhanced.
(7) The method as mentioned above, wherein the *Vibrio* bacterium is *Vibrio natriegens.*
(8) The method as mentioned above, wherein the medium contains glycerol as a carbon source.
(9) A *Vibrio* bacterium which has an ability to produce L-lysine, and has been modified so that an activity of a protein encoded by the *fucO* gene is reduced.
(10) The *Vibrio* bacterium as mentioned above, which is *Vibrio natriegens.*

### Brief Description of the Drawings

Fig. 1 shows suppression of L-lysine decomposition by *fucO* gene deficiency: (a) change of OD660 of culture medium diluted 51-fold over the period of time, (b) change of glucose concentration in the medium over the period of time, and (c) change of L-lysine concentration in the medium over the period of time. Each graph includes curves plotted with averages of results for three samples, and indicates standard deviations.
Fig. 2 shows L-lysine production from glucose by *V.*
   *natriegens* 28-15 ΔfucO/pCABD2: (a) OD660 of culture medium diluted 51-fold, (b) glucose concentration in the medium, (c) L-lysine concentration in the medium, and (d) L-lysine yield based on consumed saccharide 40 hours after the start of the culture. In the graphs, the indications of 0 to 8% NaCl mean that the graphs show averages and standard deviations of the results obtained by culture in the MS culture media containing 0 to 8% (w/v) NaCl.

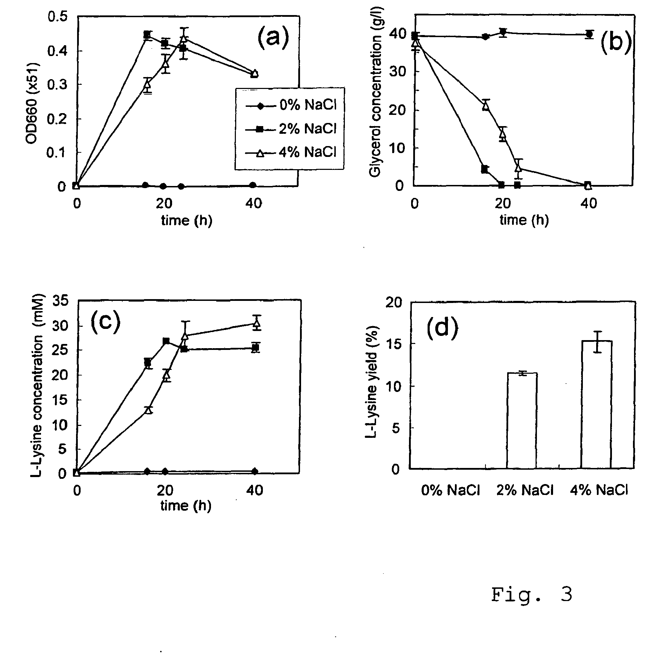

Fig. 3 shows L-lysine production from glycerol by *V.*
   *natriegens* 28-15 ΔfucO/pCABD2: (a) change of OD660 of culture medium diluted 51-fold over the period of time, (b) change of glycerol concentration in the medium over the period of time, (c) change of L-lysine concentration in the medium over the period of time, and (d) L-lysine yield based on consumed glycerol 40 hours after the start of the culture. In the graphs, the indications of 0% NaCl, 2% NaCl and 4% NaCl mean that the graphs show averages and standard deviations of the results obtained by culture in the MS culture media containing glycerol as a carbon source and 0 to 4% (w/v) NaCl.

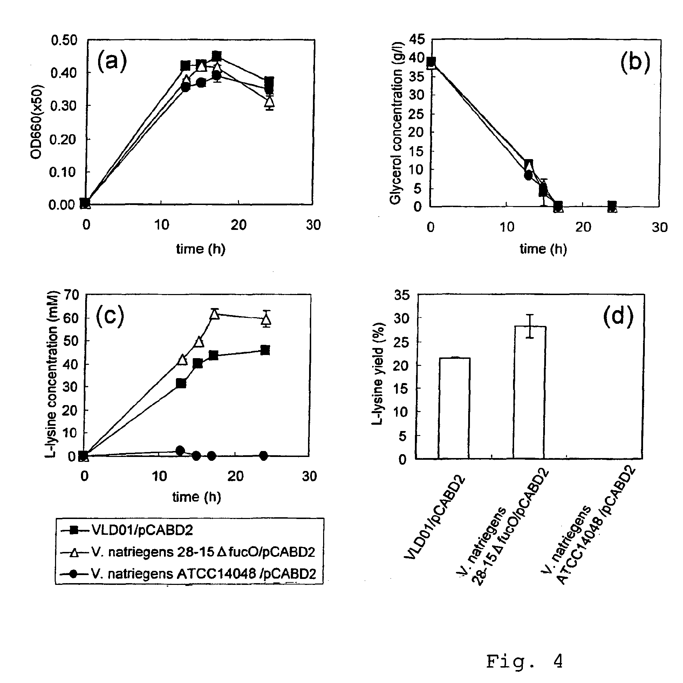

Fig. 4 shows L-lysine production from glycerol by *V. natriegens* 28-15 ΔfucO/pCABD2 and *V*. *natriegens* VLD01/pCABD2: (a) change of OD660 of culture medium diluted 50-fold, (b) change of glycerol concentration in the medium over the period of time, (c) change of L-lysine concentration in the medium over the period of time, (d) L-lysine yield based on consumed glycerol 24 hours after the start of the culture.

### Embodiments for Carrying out the Invention

### <1> Vibrio bacterium

The bacterium of the present invention is a *Vibrio* bacterium which has an ability to produce L-lysine and has been modified so that an activity of a protein encoded by the *fucO* gene is reduced.

The ability to produce L-lysine referred to herein means an ability of the bacterium of the present invention to produce and accumulate L-lysine in a medium or cells thereof in such a degree that L-lysine can be collected from the medium or cells, when the bacterium is cultured in the medium. That is, the bacterium of the present invention is a *Vibrio* bacterium that can produce L-lysine by fermentation using a saccharide or the like as a carbon source (also called direct fermentation). Specifically, when a *Vibrio* bacterium which has been modified so that the activity of the protein encoded by the *fucO* gene is reduced is cultured under preferred conditions, for example, if 1.5 times or more, preferably twice or more, more preferably 3 times or more, of L-lysine is accumulated, the bacterium has the ability to produce L-lysine.

"L-lysine" includes L-lysine of the free form and a salt thereof such as sulfate, hydrochloride and carbonate.

The *Vibrio* bacterium having the ability to produce L-lysine may be a bacterium inherently having the ability to produce L-lysine, or may be a bacterium obtained by modifying a *Vibrio* bacterium such as those described later using mutagenisis methods or DNA recombination techniquea so that the bacterium acquires the ability to produce L-lysine. The *Vibrio* bacterium of the present invention may have an ability to produce another amino acid, for example, L-threonine, L-glutamic acid, or the like, in addition to the ability to produce L-lysine.

Hereinafter, the *Vibrio* bacteria will be explained.

The *Vibrio* bacteria are facultative anaerobic gram-negative bacteria belonging to the family Vibrionaceae of γ-*Proteobacteria,* and are motile bacteria with a single polarly flagellum seen in fresh water or seawater. The *Vibrio* bacterium used in the present invention is desirably a nonpathogenic *Vibrio* bacterium, and *Vibrio* bacteria for which pathogenicity has not been known are listed in Biosafety level 1 (Biosafety in Microbiological and Biomedical Laboratories (BMBL), 4th Edition, published by Office of Health and Safety (OHS)), and such *Vibrio* bacteria as mentioned below can be used.
*Vibrio abalonicus* ATCC 27390
*Vibrio adaptatus* ATCC 19263
*Vibrio aerogenes* ATCC 700797
*Vibrio aestuarianus* ATCC 35048
*Vibrio alginolyticus* ATCC 14582
*Vibrio algosus* ATCC 14390
*Vibrio anguillarum* ATCC 43305
*Vibrio calviensis* ATCC BAA-606
*Vibrio campbellii* ATCC 25920
*Vibrio carchariae* ATCC 35084
*Vibrio coralliilyticus* ATCC BAA-450
*Vibrio costicola* ATCC 43147
*Vibrio cyclitrophicus* ATCC 700982
*Vibrio cyclosites* ATCC 14635
*Vibrio diazotrophicus* ATCC 33466
*Vibrio fischeri* ATCC 25918
*Vibrio gazogenes* ATCC 29988
*Vibrio halioticoli* ATCC 700680
*Vibrio harveyi* ATCC 14126
*Vibrio hispanica* ATCC 51589
*Vibrio ichthyoenteri* ATCC 700023
*Vibrio iliopiscarius* ATCC 51760
*Vibrio lentus* ATCC BAA-539
*Vibrio liquefaciens* ATCC 17058
*Vibrio logei* ATCC 15382
*Vibrio marinagilis* ATCC 14398
*Vibrio marinofulvus* ATCC 14395
*Vibrio marinovulgaris* ATCC 14394
*Vibrio mediterranei* ATCC 43341
*Vibrio metschnikovii* ATCC 7708
*Vibrio mytili* ATCC 51288
*Vibrio natriegens* ATCC 14048
*Vibrio navarrensis* ATCC 51183
*Vibrio nereis* ATCC 25917
*Vibrio nigripulchritudo* ATCC 27043
*Vibrio ordalii* ATCC 33509
*Vibrio orientalis* ATCC 33933
*Vibrio pectenicida* ATCC 700783
*Vibrio pelagius* ATCC 33504
*Vibrio penaeicida* ATCC 51841
*Vibrio ponticus* ATCC 14391
*Vibrio proteolyticus* ATCC 53559
*Vibrio psychroerythrus* ATCC 27364
*Vibrio salmonicida* ATCC 43839
*Vibrio shiloii* ATCC BAA-91
*Vibrio splendidus* ATCC 33125
*Vibrio tyrosinaticus* ATCC 19378
*Vibrio viscosus* ATCC BAA-105
*Vibrio wodanis* ATCC BAA-104
*Beneckea pelagia* ATCC 25916
*Listonella anguillarum* ATCC 19264
*Beneckea pelagia* and *Listonella anguillarum* closely relate to the *Vibrio* bacteria, and some strains thereof are classified as *Vibrio* bacteria according to the current classification (Thompson F.L. et al., Microbiol. Mol. Biol. Rev., 23, 403-431 (2004) and Macian M.C. et al., Syst. Appl. Microbiol., 23, 373-375 (2000)). Therefore, such bacteria can also be used in the present invention as the *Vibrio* bacterium.

Among these, it is preferable to use *Vibrio natriegens. Vibrio natriegens* is an oceanic facultative anaerobic bacterium belonging to the family Vibrionaceae of γ-*Proteobacteria,* and it was isolated on 1958 as an uronic acid-oxidizing bacterium (Payne, W.J., J. Bacteriology, 76, 301 (1958)). At first, the bacterium was considered to belong to the genus *Pseudomonas* of γ-*Proteobacteria,* but the bacterium was re-classified into the genus *Beneckea* and then incorporated into the genus *Vibrio* along with other bacteria belonging to the genus *Beneckea.* That bacterium is classified as Biosafety level 1 in ATCC, and classified as Risk Group 1 (German classification) in the German National Resource Center for Biological Material (DSMZ), and pathogenicity is not known for the bacterium.

As *Vibrio natriegens,* the *Vibrio natriegens* ATCC 14048 strain (NBRC 15636 strain) can be used.

The *Vibrio* bacteria described above are available from, for example, American Type Culture Collection (Address: P.O. Box 1549, Manassas, VA 20108, United States of America). That is, accession numbers are given to the strains, respectively, and the strains can be ordered using these accession numbers (refer to http://www.atcc.org/). The accession numbers of the strains are listed in the catalogue of the American Type Culture Collection. The *Vibrio natriegens* ATCC 14048 strain is also stored at the National Institute of Technology and Evaluation, Biological Resource Center (NITE NBRC, 2-5-8 Kazusakamatari, Kisarazushi, Chiba-ken, 292-0818, Japan), with a number of NBRC 15636, and can also be provided from that institute.

The *Vibrio* bacteria can grow well under high osmotic pressure conditions observed in a period when the produced substance is accumulated at a high level at a later stage of amino acid fermentation, or under high osmotic pressure conditions induced by a high sugar concentration, in contrast to microorganisms that have been conventionally used for production of an L-amino acid (such as *Escherichia coli* and coryneform bacteria), which cannot sufficiently grow under such conditions. The "high osmotic pressure" is, for example, not lower than 925 mOsm, preferably not lower than 1100 mOsm, more preferably not lower than 1500 mOsm. The upper limit of osmotic pressure is not particularly limited so long as the amino acid fermentation is possible, and is, for example, 2000 mOsm. In addition, the expression "the bacteria can grow well under high osmotic pressure" means that the growth rate is maintained to be, for example, not lower than 50% of the maximum growth rate at 1100 mOsm, at which the growth rate of an *E*. *coli* wild-type strain, for example MG1655 strain (ATCC 47076), decreases to 50% or less of the maximum growth rate, preferably not lower than about 90% of the maximum growth rate.

### <2> Method for imparting ability to produce L-lysine to Vibrio bacterium

A *Vibrio* bacterium having an ability to produce L-lysine can be obtained by imparting an ability to produce L-lysine to a wild-type strain of any of such *Vibrio* bacteria as described above. To impart an ability to produce L-lysine, methods conventionally employed in the breeding of coryneform bacteria, *Escherichia* bacteria etc. (see "Amino Acid Fermentation", Gakkai Shuppan Center Ltd.) can be used. Such methods include acquiring auxotrophic mutant strains, strains resistant to an analogue of L-amino acid such as L-lysine, or a metabolic regulation mutant strains, constructing a recombinant strain in which activity of an L-lysine biosynthesis enzyme is increased, and so forth. Activity of an L-lysine biosynthesis enzyme can be enhanced by increasing copy number of a gene coding for the enzyme or modifying an expression regulatory sequence such as promoter of the gene. In the breeding of L-lysine-producing bacteria, the impartation of such properties as auxotrophy, analogue resistance and metabolic regulation mutation may be combined with the enhancement of an L-lysine biosynthesis enzyme. Methods for imparting an ability to produce L-lysine will be exemplified below.

L-Lysine-producing bacteria can be bred as, for example, mutant strains that are auxotrophic for L-homoserine, or L-threonine and L-methionine (Japanese Patent Publication (KOKOKU) Nos. 48-28078 and 56-6499), mutant strains that are auxotrophic for inositol or acetic acid (Japanese Patent Laid-open Nos. 55-9784 and 56-8692), or mutant strains that are resistant to oxalysine, lysine hydroxamate, S-(2-aminoethyl)-L-cysteine, γ-methyllysine, α-chlorocaprolactam, DL-α-amino-ε-caprolactam, α-amino-lauryllactam, aspartate analogues, sulfa drugs, quinoids, or N-lauroylleucine. It is particularly preferable to breed a strain that is resistant to S-(2-aminoethyl)-L-cysteine (AEC) as an L-lysine analogue.

Examples of the mutagenesis method for obtaining a mutant strain of a *Vibrio* bacterium include ultraviolet irradiation, and treatment with a mutagen used for conventional mutagenesis such as N-methyl-N'-nitro-N-nitrosoguanidine (NTG) and nitrous acid. A *Vibrio* bacterium having an ability to produce an L-amino acid can also be obtained by selecting a naturally occurring mutant of a *Vibrio* bacterium.

An L-amino acid analogue resistant mutant strain can be obtained by, for example, inoculating *Vibrio* bacteria subjected to a mutation treatment on agar media containing the L-amino acid analogue at various concentrations and selecting a strain that forms a colony.

An auxotrophic mutant strain can be obtained by allowing *Vibrio* bacteria to form colonies on an agar medium containing a specific nutrient such as an L-amino acid, then replicating the colonies on another agar medium not containing the nutrient, and selecting a strain that cannot form a colony on the medium not containing the nutrient.

Examples of L-lysine-producing strain of *Vibrio natriegens* resistant to AEC include the *Vibrio natriegens* 28-15 strain (PERM BP-10946). This strain designated as AJ110593 strain was deposited at the National Institute of Advanced Industrial Science and Technology, International Patent Organism Depositary (Tsukuba Central 6, 1-1, Higashi 1-Chome, Tsukuba-shi, Ibaraki-ken, 305-8566, Japan) on October 24, 2006, and assigned an accession number of FERM P-21066. It was then converted to an international deposit, and assigned an accession number of FERM BP-10946.

Methods for imparting or enhancing an ability to produce L-lysine by enhancing activity of an L-lysine biosynthesis enzyme will be exemplified below.

For example, an ability to produce L-lysine can be imparted by enhancing the dihydrodipicolinate synthase activity and/or the aspartokinase activity.

Enhancement of the dihydrodipicolinate synthase activity and/or the aspartokinase activity of a *Vibrio* bacterium can be attained by constructing a recombinant DNA by ligating a gene fragment coding for dihydrodipicolinate synthase and/or a gene fragment coding for aspartokinase to a vector that functions in *Vibrio* bacteria, preferably such a vector of multi-copy type, and introducing the DNA into a host *Vibrio* bacterium for transformation. The activities of these enzymes are enhanced as a result of increase in copy numbers of the genes coding for dihydrodipicolinate synthase and/or aspartokinase in the cells of the transformant strain. Hereinafter, dihydrodipicolinate synthase may be abbreviated as DDPS, aspartokinase may be abbreviated as AK, and aspartokinase III may be abbreviated as AKIII.

Any microorganism may be used as a donor of genes coding for DDPS and AK so long as a microorganism that can express the DDPS and AK activities in a microorganism belonging to the genus *Vibrio* is chosen. The microorganism may be a wild-type strain or a mutant strain that is derived from a wild-type strain. Specific examples thereof include the *E*. *coli* (*Escherichia coli*) K-12 strain and the *Vibrio natriegens* ATCC 14048 strain (NBRC 15636). The nucleotide sequences of both genes coding for DDPS (*dapA,* Richaud, F. et al. J. Bacteriol., 297 (1986)) and AKIII (*lysC,* Cassan, M., Parsot, C., Cohen, G.N. and Patte, J.C., J. Biol. Chem., 261, 1052 (1986)) derived from an *Escherichia* bacterium have been already elucidated. Therefore, these genes can be obtained by synthesizing primers on the basis of the nucleotide sequences of the genes and performing PCR using a chromosomal DNA of a microorganism such as the *E. coli* K-12 strain or the *Vibrio natriegens* ATCC 14048 strain as a template.

Genes of *Vibrio* bacteria can also be obtained by using the following GenBank database information.
*Vibrio cholerae* O1 biovar eltor str. N16961 chromosome I, complete sequence: AE003852
*Vibrio cholerae* O1 biovar eltor str. N16961 chromosome II, complete sequence: AE003853
*Vibrio parahaemolyticus* RIMD 2210633 chromosome I, complete sequence: BA000031
*Vibrio parahaemolyticus* RIMD 2210633 chromosome II, complete sequence: BA000032
*Vibrio fischeri* ES114 chromosome I, complete sequence: CP000020
*Vibrio fischeri* ES114 chromosome II, complete sequence: CP000021
*Vibrio vulnificus* CMCP6 chromosome I, complete sequence: AE016795
*Vibrio vulnificus* CMCP6 chromosome II, complete sequence: AE016796
*Vibrio vulnificus* YJ016 chromosome I, complete sequence: BA000037
*Vibrio vulnificus* YJ016 chromosome II, complete sequence; BA000038

DDPS and AK used in the present invention are preferably not to subjcet to feedback inhibition by L-lysine. It is known that wild-type DDPS derived from a *Vibrio* bacterium is not subject to feedback inhibition by L-lysine, and wild-type AKIII derived from a *Vibrio* bacterium is subject to suppression and feedback inhibition by L-lysine. Therefore, *dapA* and *lysC* to be introduced into a *Vibrio* bacterium are preferably those coding for DDPS and AK having a mutation for desensitizing them to the feedback inhibition by L-lysine.

However, in the present invention, DDPS and AK may not necessarily be mutants. For example, it is known that DDPS derived from a *Corynebacterium* bacterium is not originally subject to the feedback inhibition by L-lysine.

Homologues of a gene coding for aspartokinase may be present, and the source of the gene is not limited so long as the gene codes for a protein having the aspartokinase activity.

For example, examples of the AK gene of *Vibrio natriegens* include the *AKO* gene, *thrA* gene, *metL* gene, *lysC* gene and putative-AK gene.

SEQ ID NO: 11 is the nucleotide sequence of the region including the *AKO* gene of *Vibrio natriegens.* It is estimated that, in SEQ ID NO: 11, GTG of the nucleotide numbers 526 to 528, GTG of the nucleotide numbers 544 to 546, or GTG of the nucleotide numbers of 568 to 570 constitutes the initiation codon, and TGA of the nucleotide numbers 1711 to 1713 constitutes the stop codon. Therefore, a DNA having the nucleotide sequence of the nucleotide numbers 526 to 1710 in SEQ ID NO: 11 (encoded amino acid sequence is that of the amino acid numbers 1 to 395 in SEQ ID NO: 12), the nucleotide sequence of the nucleotide numbers 544 to 1710 in SEQ ID NO: 11 (encoded amino acid sequence is that of the amino acid numbers 7 to 395 in SEQ ID NO: 12), or the nucleotide sequence of the nucleotide numbers 568 to 1710 in SEQ ID NO: 11 (encoded amino acid sequence is that of the amino acid numbers 15 to 395 in SEQ ID NO: 12) as an open reading frame can be used as the *AKO* gene.

SEQ ID NO: 13 is the nucleotide sequence of the region including the *thrA* gene of *Vibrio natriegens.* It is estimated that, in SEQ ID NO: 13, ATG of the nucleotide numbers 486 to 488, GTG of the nucleotide numbers 591 to 593, or GTG of the nucleotide numbers 633 to 635 constitutes the initiation codon, and TAA of the nucleotide numbers 2943 to 2945 constitutes the stop codon. Therefore, a DNA having the nucleotide sequence of the nucleotide numbers 486 to 2942 in SEQ ID NO: 13 (encoded amino acid sequence is that of the amino acid numbers 1 to 819 in SEQ ID NO: 14), the nucleotide sequence of the nucleotide numbers 591 to 2942 in SEQ ID NO: 13 (encoded amino acid sequence is that of the amino acid numbers 35 to 819 in SEQ ID NO: 14), or the nucleotide sequence of the nucleotide numbers 633 to 2942 in SEQ ID NO: 13 (encoded amino acid sequence is that of the amino acid numbers 50 to 819 in SEQ ID NO: 14) as an open reading frame can be used as the *thrA* gene.

SEQ ID NO: 15 is the nucleotide sequence of the region including the *metL* gene of *Vibrio natriegens.* It is estimated that, in SEQ ID NO: 15, ATG of the nucleotide numbers 376 to 378, GTG of the nucleotide numbers 487 to 489, or GTG of the nucleotide numbers 490 to 492 constitutes the initiation codon, and TAA of the nucleotide numbers 2782 to 2784 constitutes the stop codon. Therefore, a DNA having the nucleotide sequence of the nucleotide numbers 376 to 2781 in SEQ ID NO: 15 (encoded amino acid sequence is that of the amino acid numbers 1 to 802 in SEQ ID NO: 16), the nucleotide sequence of the nucleotide numbers 487 to 2781 in SEQ ID NO: 15 (encoded amino acid sequence is that of the amino acid numbers 38 to 802 in SEQ ID NO: 16), or the nucleotide sequence of the nucleotide numbers 490 to 2781 in SEQ ID NO: 15 (encoded amino acid sequence is that of the amino acid numbers 39 to 802 in SEQ ID NO: 16) as an open reading frame can be used as the *metL* gene.

SEQ ID NO: 17 is the nucleotide sequence of the region including the *lysC* gene of *Vibrio natriegens.* It is estimated that, in SEQ ID NO: 17, GTG of the nucleotide numbers 1060 to 1062, or ATG of the nucleotide numbers 1117 to 1119 constitutes the initiation codon, and TAA of the nucleotide numbers 2410 to 2412 constitutes the stop codon. Therefore, a DNA having the nucleotide sequence of the nucleotide numbers 1060 to 2409 in SEQ ID NO: 17 (encoded amino acid sequence is that of the amino acid numbers 1 to 450 in SEQ ID NO: 18), or the nucleotide sequence of the nucleotide numbers 1117 to 2409 in SEQ ID NO: 17 (encoded amino acid sequence is that of the amino acid numbers 20 to 450 in SEQ ID NO: 18) as an open reading frame can be used as the *lysC* gene.

SEQ ID NO: 19 is the nucleotide sequence of the region including a putative-AK gene of *Vibrio natriegens.* It is estimated that, in SEQ ID NO: 19, ATG of the nucleotide numbers 344 to 346, ATG of the nucleotide numbers 380 to 382, or ATG of the nucleotide numbers 470 to 472 constitutes the initiation codon, and TAA of the nucleotide numbers 1766 to 1768 constitutes the stop codon. Therefore, a DNA having the nucleotide sequence of the nucleotide numbers 344 to 1765 in SEQ ID NO: 19 (encoded amino acid sequence is that of the amino acid numbers 1 to 474 of SEQ ID NO: 20), the nucleotide sequence of the nucleotide numbers 380 to 1765 in SEQ ID NO: 19 (encoded amino acid sequence is that of the amino acid numbers 13 to 474 in SEQ ID NO: 20), or the nucleotide sequence of the nucleotide numbers 470 to 1765 of SEQ ID NO: 19 (encoded amino acid sequence is that of the amino acid numbers 43 to 474 in SEQ ID NO: 20) as an open reading frame can be used as the putative-AK gene.

The aforementioned *AKO* gene, *thrA* gene, *metL* gene, *lysC* gene and putative-AK gene may be a DNA hybridizable with a complementary strand of each sequence under stringent conditions and coding for a protein having the aspartokinase activity.

The "stringent conditions" referred to herein are conditions where a so-called specific hybrid is formed, and a non-specific hybrid is not formed. Although it is difficult to definitely define these conditions with numerals, examples of the stringent conditions include those where highly homologous DNAs hybridize each other, for example, DNAs not less than 80% homologous, preferably not less than 90% homologous, more preferably not less than 95% homologous, still more preferably not less than 97% homologous, particularly preferably not less than 99% homologous, hybridize each other, and DNAs less homologous than the above do not hybridize each other, or conditions of washing once, preferably 2 or 3 times, at salt concentrations and temperature corresponding to washing in typical Southern hybridization, i.e., 1 x SSC, 0.1% SDS at 60°C, preferably 0.1 x SSC, 0.1% SDS at 60°C, more preferably 0.1 x SSC, 0.1% SDS at 68°C. The stringent conditions used in the present invention are washing conditions of 1 x SSC, 0.1% SDS at 60°C.

In this specification, the term "homology" may means "identity".

The aspartokinase activity can be measured by the method described in Miyajima, R. et al., The Journal of Biochemistry, 63 (2), 139-148 (1968).

The aforementioned *AKO* gene, *thrA* gene, *metL* gene, *lysC* gene and putative-AK gene are not limited to wild-type genes and may be mutant or artificially modified genes coding for a conservative variant protein having the amino acid sequence encoded by the open reading frame of each gene, but including substitutions, deletions, insertions, additions or the like of one or several amino acids at one or a plurality of positions, so long as the genes code for a protein having the aspartokinase activity.

Although the number of the "one or several" amino acid residues referred to herein may differ depending on the position in the three-dimensional structure or the types of amino acid residues of the protein, specifically, it may be preferably 1 to 20, more preferably 1 to 10, still more preferably 1 to 5. The aforementioned substitutions, deletions, insertions, or additions of one or several amino acid residues is a conservative mutation that preserves the normal function of the protein. The conservative mutation is a mutation wherein substitution takes place mutually among Phe, Trp, and Tyr, if the substitution site is an aromatic amino acid; among Leu, Ile and Val, if it is a hydrophobic amino acid; between Gln and Asn, if it is a polar amino acid; among Lys, Arg and His, if it is a basic amino acid; between Asp and Glu, if it is an acidic amino acid; and between Ser and Thr, if it is an amino acid having a hydroxyl group. Typical examples of a conservative mutation are conservative substitutions, and substitutions considered conservative substitutions include, specifically, substitution of Ser or Thr for Ala, substitution of Gln, His or Lys for Arg, substitution of Glu, Gln, Lys, His or Asp for Asn, substitution of Asn, Glu or Gln for Asp, substitution of Ser or Ala for Cys, substitution of Asn, Glu, Lys, His, Asp or Arg for Gln, substitution of Gly, Asn, Gln, Lys or Asp for Glu, substitution of Pro for Gly, substitution of Asn, Lys, Gln, Arg or Tyr for His, substitution of Leu, Met, Val or Phe for Ile, substitution of Ile, Met, Val or Phe for Leu, substitution of Asn, Glu, Gln, His or Arg for Lys, substitution of Ile, Leu, Val or Phe for Met, substitution of Trp, Tyr, Met, Ile or Leu for Phe, substitution of Thr or Ala for Ser, substitution of Ser or Ala for Thr, substitution of Phe or Tyr for Trp, substitution of His, Phe or Trp for Tyr, and substitution of Met, Ile or Leu for Val. The aforementioned amino acid substitutions, deletions, insertions, additions, inversions or the like may be a result of a naturally-occurring mutation or a variation due to an individual difference or difference of species of a microorganism which harbors the AKO, *thrA, metL, lysC* and putative-AK genes. Such a mutant or modified gene as described above can be obtained by modifying the nucleotide sequence of each open reading frame described above by, for example, site-specific mutagenesis so that the encoded protein includes substitution, deletion, insertion or addition of amino acid residues at a specific position.

As the AKO gene, *thrA* gene, *metL* gene, *lysC* gene and putative-AK gene, there may be used genes that code for an amino acid sequence not less than 80% homologous, preferably not less than 90% homologous, more preferably not less than 95% homologous, still more preferably not less than 97% homologous, particularly preferably not less than 99% homologous, to the entire amino acid sequence encoded by the aforementioned open reading frame of each gene, and code for a protein having the aspartokinase activity. The homology of amino acid sequences and nucleotide sequences may be determined by using, for example, the algorithm BLAST (Proc. Natl. Acad. Sci. USA, 90, 5873 (1993)) created by Karlin and Altschul or FASTA (Methods Enzymol., 183, 63 (1990)). On the basis of the algorithm BLAST, the programs called BLASTN and BLASTX have been developed (refer to http://www.ncbi.nlm.nih.gov).

The above descriptions concerning conservative variants and genes coding for them, as well as conservative mutation are also applied to enzymes other than aspartokinase and genes coding for them, as well as the FucO protein and the *fucO* gene described later.

Plasmids to be used for gene cloning may be those replicable in bacteria such as *Escherichia* bacteria, and specific examples thereof include pBR322, pTWV228, pMW119, pUC 19, and so forth.

As the vector that functions in *Vibrio* bacteria, any vector autonomously replicable in *Vibrio* bacteria may be used. As the vector plasmid, any of vector plasmids having ori derived from pUC plasmid, pACYC184 plasmid, or IncQ plasmid may be used. As the marker gene used for the selection, the kanamycin resistance gene derived from Tn903, the chloramphenicol resistance gene derived from Tn9, a streptomycin resistant gene, a tetracycline resistant gene and so forth may be used.

In order to prepare a recombinant DNA by ligating *dapA* and *lysC* to a vector that functions in *Vibrio* bacteria, the vector is digested with restriction enzymes that correspond to the terminuses of a DNA fragment containing *dapA* and *lysC.* Ligation is usually performed by using a ligase such as T4 DNA ligase. *dapA* and *lysC* may be carried by separate vectors or a single vector.

Examples of DNA coding for a mutant dihydrodipicolinate synthetase which is not subject to feedback inhibition by L-lysine include a DNA coding for such a protein having an amino acid sequence in which the histidine residue at position 118 is replaced by tyrosine residue. Examples of DNA coding for a mutant aspartokinase which is not subject to feedback inhibition by L-lysine include a DNA coding for an AKIII having an amino acid sequence in which the threonine residue at position 352, the glycine residue at position 323, and the methionine residue at position 318 are replaced by isoleucine, asparagine and isoleucine residues, respectively (for these mutants, see U.S. Patent Nos. 5,661,012 and 6,040,160). Such mutant DNAs can be obtained by site-specific mutagenesis using PCR or the like.

Wide host-range plasmids RSFD80, pCAB1, and pCABD2 are known as plasmids containing a mutant *dapA* gene coding for a mutant dihydrodipicolinate synthase and a mutant *lysC* gene coding for a mutant aspartokinase (U.S. Patent No. 6,040,160). *Escherichia coli* JM109 strain transformed with the plasmid was named AJ12396 (U.S. Patent No. 6,040,160), and the strain was deposited at the National Institute of Bioscience and Human-Technology, Agency of Industrial Science and Technology, Ministry of International Trade and Industry (currently National Institute of Advanced Industrial Science and Technology, International Patent Organism Depositary) on October 28, 1993 and assigned an accession number of FERM P-13936. The deposit was then converted to an international deposit under the provisions of Budapest Treaty on November 1, 1994 and assigned an accession number of FERM BP-4859. RSFD80 can be obtained from the AJ12396 strain by a conventional method.

The recombinant DNA prepared as described above may be introduced into a *Vibrio* bacterium by any method that affords sufficient transformation efficiency, and examples thereof include electroporation (Canadian Journal of Microbiology, 43, 197 (1997)).

The DDPS activity and/or the AK activity can also be enhanced by introducing multiple copies of the *dapA* and/or *lysC* into a chromosomal DNA of a *Vibrio* bacterium. Introduction of multiple copies of the *dapA* and/or *lysC* into a chromosomal DNA of a *Vibrio* bacterium can be attained by homologous recombination which targets a sequence present on the chromosomal DNA in multiple copies. Such a sequence present on a chromosomal DNA in multiple copies may be a repetitive DNA or an inverted repeat present on the end of a transposable element. Alternatively, as disclosed in Japanese Patent Laid-open No. 2-109985, multiple copies of *dapA* and/or *lysC* can be introduced into the chromosomal DNA by inserting the genes into a transposon and transferring it so that multiple copies of the genes are introduced into the chromosomal DNA. These methods increase the copy numbers of *dapA* and/or *lysC,* which leads to enhancement in the DDPS activity and/or the AK activity.

Besides the above-described gene amplification, the enhancement of the DDPS activity and/or the AK activity can also be attained by replacing an expression regulatory sequence such as a promoter of *dapA* and/or *lysC* with a potent sequence (Japanese Patent Laid-open No. 1-215280). Examples of potent promoters include *lac* promoter, trp promoter, trc promoter, tac promoter, PR promoter and PL promoter of lambda phage, hybrid promoters of these, and so forth. Replacement with these promoters enhances the expression of *dapA* and/or *lysC,* and the DDPS activity and/or the AK activity are thereby increased. Replacement of an expression regulatory sequence may be combined with increase of the copy number of *dapA* and/or *lysC* (Science, 1997 Sep. 5, 277 (5331):1453-74; Nucleic Acids Res., 1993 Apr. 11, 21(7):1507-16; Proc. Natl. Acad. Sci. USA, 1983 Jan., 80(1):21-5, International Patent Publications WO98/04715, WO98/17806, U.S. Patent Nos. 5,830,690, and 5,861,273).

Digestion, ligation, etc. of DNA, preparation of chromosomal DNA, PCR, preparation of plasmid DNA, transformation, design of oligonucleotides to be used as primers and so forth can be performed according to conventional methods well known to those skilled in the art. Such methods are described in Sambrook, J., Fritsch, E.F., and Maniatis, T., "Molecular Cloning A Laboratory Manual, Second Edition", Cold Spring Harbor Laboratory Press, (1989) and so forth.

In addition to enhancement of the DDPS activity and/or the AK activity, activities of other enzymes involved in L-lysine biosynthesis may be enhanced. Examples of such enzymes include enzymes in the diaminopimelic acid pathway such as dihydrodipicolinate reductase (*dapB*)*,* diaminopimelate decarboxylase (*lysA*)*,* diaminopimelate dehydrogenase (*ddh*) (International Patent Publication WO96/40934 for those mentioned above), phosphoenolpyruvate carboxylase (*ppc*, Japanese Patent Laid-open No. 60-87788), aspartate aminotransferase (*aspC,* Japanese Patent Publication No. 6-102028), diaminopimelate epimerase (*dapF,* Japanese Patent Laid-open No. 2003-135066), and aspartate semialdehyde dehydrogenase (*asd,* International Patent Publication WO00/61723); and genes coding for enzymes in the aminoadipic acid pathway such as homoaconitate hydratase (Japanese Patent Laid-open No. 2000-157276). Those mentioned in the parentheses after the enzyme names are gene names (the same shall apply to the following descriptions).

The plasmid pCABD2 contains a mutant *dapA* gene derived from *Escherichia coli* and coding for DDPS having a mutation for desensitization to the feedback inhibition by L-lysine, a mutant *lysC* gene derived from *Escherichia coli* and coding for AKIII having a mutation for desensitization to the feedback inhibition by L-lysine, the *dapB* gene derived from *Escherichia coli* and coding for dihydrodipicolinate reductase, and the *ddh* gene derived from *Brevibacterium lactofermentum* and coding for diaminopimelate dehydrogenase (International Patent Publications WO95/16042 and WO01/53459).

The *Vibrio* bacterium of the present invention may be a bacterium in which the ability to produce L-lysine is increased by enhancing L-lysine secretion activity. For example, by increasing expression amount of the *ybjE* gene, or increasing expression amount of the *lysE* gene, the L-lysine secretion activity can be enhanced (Japanese Patent Laid-open No. 2005-237379, International Patent Publication WO97/23697).

Furthermore, in the bacterium of the present invention, activity of an enzyme that catalyzes a reaction branching off from the L-lysine biosynthesis pathway to produce a compound other than L-lysine, or activity of an enzyme that negatively functions against L-lysine synthesis or accumulation may be decreased or deleted. Examples of such an enzyme for the L-lysine production include homoserine dehydrogenase, malic enzyme, and so forth, and strains in which activities of such enzymes are decreased or deficient can be constructed by referring to International Patent Publications WO95/23864, WO96/17930, and WO2005/010175.

Activities of these enzymes can be decreased or deleted by introducing such a mutation that the activities of the enzymes in a cell are decreased or deleted into the genes coding for the enzymes on the genome using a conventional mutagenesis or genetic engineering techniques. Such introduction of a mutation can be attained by, for example, eliminating the genes coding for the enzymes on the genome by gene recombination or by modifying an expression regulatory sequence such as a promoter or Shine-Dalgamo (SD) sequence. In addition, it can also be attained by introducing a mutation for an amino acid substitution (missense mutation) into the region coding for the enzymes on the genome, introducing a stop codon (nonsense mutation), introducing a frame shift mutation that adds or deletes one or two nucleotides, or deleting a part or whole region of the gene (Journal of Biological Chemistry, 272, 8611-8617 (1997); Proc. Natl. Acad. Sci. USA, 95, 5511-5515 (1998), J. Biol. Chem., 266, 20833-20839 (1991)). Furthermore, the activities of the enzymes can also be decreased or eliminated by constructing a mutant gene coding for a mutant enzyme a part or all of which coding region is deleted, and then replacing the normal gene on the genome with the mutant gene by homologous recombination or the like, or introducing a transposon or an IS factor into the gene.

For example, such a method as described below is used to introduce a mutation that decreases or eliminates the activities of the above-mentioned enzymes by genetic recombination. A mutant gene is prepared by modifying a partial sequence of a target gene so that it does not produce an enzyme that can function normally. Then, a *Vibrio* bacterium is transformed with a DNA containing the mutant gene to cause recombination of a gene on the genome with the mutant gene, and thereby the target gene on the genome is replaced with the mutant gene. Examples of this type of gene substitution using homologous recombination include the method using a linear DNA such as the method called "Red-driven integration" (Datsenko, K.A, and Wanner, B.L., Proc. Natl. Acad. Sci. USA, 97:6640-6645 (2000), and a method consisting of a combination of the Red-driven integration and an excision system derived from A phage (Cho, E.H., Gumport, R.I., Gardner, J.F., J. Bacteriol., 184, 5200-5203 (2002), refer to International Patent Publication WO2003/010175) a method using a plasmid containing a temperature-sensitive replication origin (U.S. Patent No. 6,303,383; Japanese Patent Laid-open No. 05-007491), and so forth. Such site-specific mutation as mentioned above based on gene substitution utilizing homologous recombination can also be performed by using a plasmid that is not able to replicate in a host cell.

The aforementioned method for reducing enzyme activity can also be applied to decrease of the activity of the FucO protein described below.

### <3> Decrease of activity of protein encoded by fucO gene

The bacterium of the present invention can be obtained by modifying such a bacterium belonging to the genus *Vibrio* and having an ability to produce L-lysine as described above so that activity of the protein encoded by the *fucO* gene (hereafter the protein is also described as "FucO") is reduced. In breeding of the *Vibrio* bacterium of the present invention, either the impartation of the ability to produce L-lysine or the modification for reducing the activity of the FucO protein may be performed first. Although the bacterium of the present invention may be a bacterium which has been modified so that the activity of the protein encoded by the *fucO* gene is reduced as compared to a wild-type strain or a non-modified strain, it is more desirable that it further has improved ability to accumulate L-lysine as compared to a wild-type strain or a non-modified strain.

In the present invention, the *fucO* gene means a homologue of the *fucO* gene of *E*. *coli* obtainable from a *Vibrio* bacterium, and examples include, for example, a gene having the nucleotide sequence of SEQ ID NO: 1. The nucleotide sequence of SEQ ID NO: 1 is the nucleotide sequence of the *fucO* gene of the *V. natriegens* 28-15 strain. The amino acid sequence encoded by this gene is shown in SEQ ID NO: 2.

The *fucO* gene of the present invention is a gene showing homology to the *fucO* gene of *E*. *coli.* The *fucO* gene of the *E*. *coli* MG1655 strain is registered at GenBank as a gene coding for L-1,2-propanediol oxidoreductase (complementary strand of GenBank NC_000913.2, nucleotide numbers 2929887 to 2931038).

Moreover, the *fucO* gene of the present invention includes the *eutG* gene of *Vibrio parahaemolyticus* and a gene showing homology to that gene. The *eutG* gene product of the *V. parahaemolyticus* RIMD 2210633 strain is registered at the GenPept database (http://www.ncbi.nlm.nih.gov/protein/NP_797614.1?report=gen pept) as an iron-containing alcohol dehydrogenase (NP_797614). Moreover, the *eutG* gene is registered at GenBank as a gene of iron-containing alcohol dehydrogenase (NC_004603.1, nucleotide numbers 1301840 to 1303159).

The homology between the *fucO* gene product of *E*. *coli* and the *eutG* gene product of *E*. *coli* in terms of amino acid composition is 77.3% (analyzed by using Genetix ver. 9.0.3 produced by Molecular Devices, Inc.).

A homologue of the *fucO* gene of a *Vibrio* bacterium can be obtained by, for example, searching for a gene showing high homology to a gene having the nucleotide sequence of SEQ ID NO: 1, the *fucO* gene of *E*. *coli,* or the *eutG* gene using BLAST (http://blast.genome.jp/). Degree of the homology is not particularly limited so long as accumulation of L-lysine is improved by reduction of the activity of the expression product of the gene, but the homology is, for example, 80% or more, preferably 90% or more, more preferably 95% or more, still more preferably 97% or more, particularly preferably 99% or more, with respect to the entire amino acid sequence.

Examples of the protein encoded by the *fucO* gene include those having the amino acid sequence of SEQ ID NO: 2. However, the protein may be a conservative variant so long as the function of the protein is not changed.

Furthermore, the *fucO* gene may be a DNA hybridizable with a DNA complementary to the nucleotide sequence of SEQ ID NO: 1 under stringent conditions, so long as it codes for the FucO protein. The "stringent conditions" have the same meaning as that described above.

The expression "activity of the protein encoded by the *fucO* gene is reduced" means that the function of the FucO protein encoded by the *fucO* gene is attenuated or deleted due to introduction of a mutation into the coding region of the *fucO* gene attained by using a drug or genetic engineering, and also means that expression or translation of the *fucO* gene is reduced due to introduction of a mutation into an expression control region of the *fucO* gene or the like, and thus amount of the FucO protein is reduced. Furthermore, the activity of the FucO protein can be reduced by the so-called gene disruption, i.e., deletion of a part of the *fucO* gene or the entire *fucO* gene on a chromosome. Reduction of the activity includes complete elimination of the activity.

In order to reduce the activity of the FucO protein, specifically, the methods for reducing an enzyme activity described for impartation of ability to produce L-lysine can be used.

### <4> Method for producing L-lysine

The method of the present invention is a method of culturing the bacterium of the present invention in a medium to produce and accumulate L-lysine in the medium or cells of the bacterium, and collecting L-lysine from the medium or the cells.

As the medium to be used, media conventionally used for the production of L-amino acids by fermentation using microorganisms can be used. That is, usual media containing a carbon source, a nitrogen source, inorganic ions, and optionally other organic components as required can be used. As the carbon source, saccharides such as glucose, sucrose, lactose, galactose, fructose, and hydrolysates of starches; alcohols such as glycerol and sorbitol; and organic acids such as fumaric acid, citric acid and succinic acid can be used. In the present invention, it is especially preferable to use glycerol as the carbon source.

In the present invention, it is preferable to use glycerol as the carbon source. Glycerol may be used at any concentration so long as a concentration suitable for production of L-lysine is chosen. When glycerol is used as a sole carbon source in the medium, it is preferably contained in the medium in an amount of about 0.1 to 50 w/v %, more preferably about 0.5 to 40 w/v %, particularly preferably about 1 to 30% w/v %. Glycerol can also be used in combination with other carbon sources such as glucose, fructose, sucrose, blackstrap molasses and starch hydrolysate. In this case, although glycerol and other carbon sources may be mixed at an arbitrary ratio, it is desirable that the ratio of glycerol in the carbon source is 10% by weight or more, more preferably 50% by weight or more, still more preferably 70% by weight or more. Preferable other carbon sources are saccharides such as glucose, fructose, sucrose, lactose, galactose, blackstrap molasses, starch hydrolysate and a sugar solution obtained by hydrolysis of biomass, alcohols such as ethanol, and organic acids such as fumaric acid, citric acid and succinic acid. Among these, glucose is preferred.

Although initial concentration of glycerol at the time of starting the culture is preferably as described above, glycerol may be supplemented with consumption of glycerol during the culture.

Glycerol to be used may be pure glycerol or crude glycerol. Crude glycerol refers to industrially produced glycerol containing impurities. Crude glycerol is industrially produced by contacting fats and oils with water under a high temperature and high pressure to hydrolyze them, or by the esterification reaction for biodiesel fuel production. Biodiesel fuel refers to aliphatic acid methyl esters produced from fats and oils and methanol by a transesterification reaction, and crude glycerol is produced as a by-product of this reaction (refer to Fukuda, H., Kondo, A., and Noda, H., 2001, J. Biosci. Bioeng., 92, 405-416). In the biodiesel fuel production process, the alkaline catalyst method is used for the transesterification in many cases, and acids are added for neutralization. Therefore, crude glycerol of a purity of about 70 to 95% by weight, containing water and impurities, is produced. Crude glycerol produced in the biodiesel fuel production process contains, in addition to water, residual methanol, salts of alkali such as NaOH as a catalyst and an acid used for neutralizing the alkali, such as K₂SO₄, as impurities. Although it depends on manufacturers and production methods, the amount of these salts and methanol can reach several percent. The crude glycerol preferably contains ions derived from the alkali and the acid used for neutralization thereof, such as sodium ions, potassium ions, chloride ions, and sulfate ions, in an amount of 2 to 7%, preferably 3 to 6%, more preferably 4 to 5.8%, based on the weight of the crude glycerol. Although methanol may not be contained as an impurity, it is preferably present in an amount of 0.01% or less.

The crude glycerol may further contain trace amounts of metals, organic acids, phosphorus, aliphatic acids, and so forth. Examples of the organic acids include formic acid, acetic acid, and so forth, and although they may not be contained as impurities, they are preferably present in an amount of 0.01% or less. As the trace metals contained in the crude glycerol, trace metals required for growth of the microorganism are preferred, and examples include, for example, magnesium, iron, calcium, manganese, copper, zinc, and so forth. Magnesium, iron, and calcium are preferably present in an amount of 0.00001 to 0.1%, preferably 0.0005 to 0.1%, more preferably 0.004 to 0.05%, still more preferably 0.007 to 0.01%, in terms of the total amount based on the weight of the crude glycerol. Manganese, copper, and zinc are preferably present in an amount of 0.000005 to 0.01%, preferably 0.000007 to 0.005%, more preferably 0.00001 to 0.001%, in terms of the total amount.

The purity of the crude glycerol may be 10% or higher, preferably 50% or higher, more preferably 70% or higher, particularly preferably 80% or higher. As long as the impurities are within the aforementioned range, the purity of the glycerol may be 90% or higher.

When the crude glycerol is used, the crude glycerol may be added to the medium according to the purity of the glycerol so that the amount of glycerol is within the concentration range described above. Both glycerol and the crude glycerol may be added to the medium.

As the nitrogen source, inorganic ammonium salts such as ammonium sulfate, ammonium chloride, and ammonium phosphate, organic nitrogen such as soybean hydrolysate, ammonia gas, aqueous ammonia, and so forth can be used. As organic trace nutrient sources, the medium desirably contains required substances such as vitamin B1 and L-homoserine, yeast extract, and so forth in appropriate amounts. Other than the above, potassium phosphate, magnesium sulfate, iron ions, manganese ions and so forth are added in small amounts, as required. In addition, the medium used for the present invention may be either a natural medium or a synthetic medium, so long as a medium containing a carbon source, a nitrogen source, and inorganic ions, and containing other organic trace ingredients as required is used.

The culture is preferably performed for 1 to 7 days under aerobic conditions. The culture temperature is preferably 24 to 37°C, and pH during the culture is preferably 5 to 9. For pH adjustment, inorganic or organic acidic or alkaline substances, ammonia gas, and so forth can be used.

When a basic amino acid such as L-lysine is produced, the production can be performed by a method in which fermentation is performed by controlling pH of the medium during the culture to be 6.5 to 9.0, and the pH of the medium at the end of the culture to be 7.2 to 9.0, and providing a culture period when the medium contains 20 mM or more of bicarbonate ions and/or carbonate ions, so that these bicarbonate ions and/or carbonate ions serve as counter ions of the basic amino acid, and the objective basic amino acid is then collected (Japanese Patent Laid-open No. 2002-65287, U.S. Patent Published Application No. 2002/0025564, EP 1813677 A).

When a microorganism having a basic amino acid-producing ability is cultured in a medium under aerobic conditions, carbonate ions, bicarbonate ions, or both can be used as major counter ions of the basic amino acid. To provide carbonate ions and/or bicarbonate ions in the medium in an amount required to serve as counter ions of the basic amino acid, it is known that the pH of the medium can be controlled to be 6.5 to 9.0, or 6.5 to 8.0 in another example, during the culture, and can be 7.2 to 9.0 at the end of the culture, and the pressure in the fermentation tank can be controlled so that it is positive during fermentation, or carbon dioxide or a mixed gas containing carbon dioxide can be supplied into the medium (Japanese Patent Laid-open No. 2002-65287, U.S. Patent Published Application No. 2002/0025564, EP 1813677 A).

L-lysine can be collected from a fermentation solution by a combination of the following conventionally known methods: use of an ion-exchange resin (Nagai, H. et al., Separation Science and Technology, 39(16), 3691-3710), precipitation, membrane separation (Japanese Patent Laid-open Nos. 9-164323 and 9-173792), crystallization (International Patent Publications WO2008/078448, WO2008/078646), and so forth. When L-lysine is accumulated in cells, the cells can be disrupted with, for example, ultrasonication or the like, and L-lysine can be collected by the ion exchange resin method or the like from supernatant obtained by removing the cells from the cell-disrupted suspension by centrifugation.

The collected L-lysine may contain bacterial cells, medium components, moisture, and by-product metabolites of the bacterium in addition to L-lysine. Purity of the collected L-lysine can be 50% or higher, 85% or higher, or even 95% or higher (Japanese Patent No. 1214636, U.S. Patent Nos. 5,431,933, 4,956,471, 4,777,051, 4,946,654, 5,840,358, 6,238,714, U.S. Patent Published Application No. 2005/0025878).

Furthermore, when L-lysine precipitates in the medium, it can be collected by centrifugation, filtration or the like. L-Lysine precipitated in the medium and L-lysine dissolved in the medium may be isolated together after the L-lysine dissolved in the medium is crystallized.

### Examples

Hereinafter, the present invention will be still more specifically explained with reference to examples.

### Example 1: Construction of fucO gene-disrupted V.

### natriegens

The *fucO* gene on the chromosome of lysine-producing bacterium, *V. natriegens* 28-15, was disrupted.

First, the *sacB* gene of *Bacillus subtilis* was amplified by PCR using pDNR-Dual Doner vector (Clontech, USA) having the *sacB* gene of *Bacillus subtilis* as a template DNA and synthetic DNAs having the sequences of SEQ ID NOS: 3 and 4 as primers. The *sacB* gene is a gene coding for levan sucrase, and used for efficiently selecting a strain in which a vector portion is eliminated from a chromosome (Schafer, A. et al., Gene, 145, 69-73 (1994)). That is, if levan sucrase is expressed, sucrose is assimilated, and the produced levan lethally acts on bacteria, so that they cannot grow. Therefore, if a strain in which a vector carrying a levan sucrase gene still remains on a chromosome is cultured on a plate containing sucrose, it cannot grow, and thus only a strain in which the vector is eliminated can be selected on the plate containing sucrose.

The amplified *sacB* gene fragment and pUT399 were digested with the restriction enzyme EcoRI and ligated to obtain a vector pUT_sacB. pUT399 used here is a plasmid having the replication origin of R6K, and containing the *mob* region required for conjugative transfer, and it is a plasmid that cannot replicate in a strain not having the *pir* gene (U.S. Patent No. 7,090,998). Then, PCR was performed by using the chromosomal DNA of *V. natriegens* 28-15 as a template DNA and the synthetic DNAs having the sequences of SEQ ID NOS: 5 and 6 or SEQ ID NOS: 7 and 8 as primers, sequences of about 800 bp on both sides of the *fucO* gene were amplified, respectively. Then, PCR was performed again by using a mixture of two kinds of the obtained PCR products as a template DNA, and synthetic DNAs of the sequences of SEQ ID NOS: 5 and 7 as primers. The obtained PCR product and pUT_sacB were digested with the restriction enzymes *Sal*I and *Sph*I, and ligated to obtain a vector pUT_sacB_fucOFR. pUT_sacB_fucOFR is a plasmid for disrupting the *fucO* gene, lacking a part of the coding region of *fucO.* And pUT_sacB_fucOFR was introduced into the *E. coli* S17-1 (λpir+, available from Biomedal, R. Simon., et al., Bio/Technology, 1:784-791 (1983)) to obtain S17-1/pUT_sacB_fucOFR.

Then, S17-1/pUT_sacB_fucOFR was inoculated into 1.5 ml of the LB medium (containing 10 g/L of Bacto tryptone, 5 g/L of Bacto yeast extract, 5 g/L of NaCl, and 40 mg/L of chloramphenicol, pH 7.0), and cultured at 37°C for 18 hours. The cells were collected by centrifuging the obtained culture medium (at 5000 rpm for 5 minutes), and suspended in 50 µl of fresh LB medium. This cell suspension was inoculated on the LB-NaCl agar medium (containing 10 g/L of Bacto tryptone, 5 g/L of Bacto yeast extract, 30 g/L of NaCl, 0.4 g/L of MgSO₄ and 20 g/L of agar, pH 7.0) in the shape of circle having a diameter of 2 cm, and dried by leaving the inoculated medium at room temperature for 30 minutes. In parallel, *V. natriegens* 28-15 was inoculated in 1.5 ml of the LB-NaCl medium (containing 10 g/L of Bacto tryptone, 5 g/L of Bacto yeast extract, 30 g/L of NaCl, and 0.4 g/L of MgSO₄, pH 7.0), and cultured at 37°C for 18 hours. The cells were collected by centrifuging the obtained culture medium (at 6000 rpm for 2 minutes), and suspended in 50 µl of fresh LB-NaCl medium. This cell suspension was inoculated on the site at which S17-1/pUT_sacB_fucOFR had been previously inoculated in the shape of circle, and dried by leaving the inoculated medium at room temperature for 30 minutes. Then, the cells were left at 37°C for 4 hours to transfer pUT_sacB_fucOFR harbored by S17-1/pUT_sacB_fucOFR to *V. natriegens* 28-15 by conjugative transfer.

The obtained bacterial cells were scraped together, and suspended in 1 ml of the LB-NaCl medium, then the total volume of the suspension was inoculated on a TCBS agar medium (containing 5 g/L of yeast extract, 10 g/L of peptone, 17 g/L of sucrose, 10 g/L of sodium thiosulfate (pentahydrate), 10 g/L of sodium citrate (dihydrate), 3 g/L of sodium cholate, 1 g/L of ferric citrate, 10 g/L of sodium chloride, 5 g/L of bovine bile powder, 0.04 g/L of bromothymol blue, 0.05 g/L of thymol blue, 15 g/L of agar, and 10 mg/L of chloramphenicol, NISSUI PHARMACEUTICAL CO., LTD., Japan), and culture was performed at 37°C for 18 hours to allow formation of yellow colonies. On the TCBS medium, *E*. *coli* cannot proliferate. Furthermore, pUT_sacB_fucOFR cannot replicate in *V. natriegens* 28-15. Therefore, the obtained colonies were those of a *V. natriegens* 28-15 strain that acquired chloramphenicol resistance by introduction of pUT_sacB_fucOFR into the chromosome.

That strain was inoculated in the LB-NaCl medium supplemented with 10% (w/v) sucrose, and formation of colonies was allowed to obtain *V. natriegens* 28-15 in which the *fucO* gene on the chromosome was disrupted with elimination of pUT_sacB_fucOFR inserted into the chromosome (*V. natriegens* 28-15 ΔfucO). The disruption of the *fucO* gene was confirmed by PCR using chromosomal DNA extracted from the obtained strain in a conventional manner as a template DNA, and DNAs having the sequences of SEQ ID NOS: 9 and 10 as primers.

### Example 2

L-Lysine decomposition activity of *V. natriegens* 28-15 ΔfucO was examined.

pCABD2 (U.S. Patent No. 6,040,160) was introduced into *V. natriegens* 28-15 ΔfucO and *V. natriegens* 28-15 by electroporation. Electroporation was performed by using Gene Pluser Xcell (BioRad, USA) with pulse conditions of 9 kV/cm, 25 µF, and 200 Q.

The obtained *V. natriegens* 28-15 ΔfucO/pCABD2 and *V. natriegens* 28-15/pCABD2 were each cultured on the LB-NaCl agar medium (containing 500 mg/L of streptomycin) at 37°C for 10 hours. The obtained cells were scraped together, inoculated into 20 ml of the MS medium (containing 500 mg/L of streptomycin) contained in a Sakaguchi flask (volume: 500 ml) at OD660 of 0.1, and cultured at 37°C with shaking. Each strain was cultured in three flasks. The medium was periodically sampled in a volume of 1 ml, and OD660 as well as glucose concentration and L-lysine concentration in the medium were measured. OD660 was measured for the culture medium diluted with 51 times with a 2% (w/v) NaCl aqueous solution by using a spectrophotometer DU-800 (Beckman Coulter, USA). The glucose concentration and L-lysine concentration in the medium were measured by using Biotech Analyzer AS-300 (Sakura Seiki Co., Ltd., Japan).

### [MS culture medium]

### (Final concentration)

| | |
|---|---|
| Glucose | 40 g/L (separately sterilized) |
| MgSO₄·7H₂O | 1 g/L (separately sterilized) |
| (NH₄)₂SO₄ | 16 g/L |
| KH₂PO₄ | 1 g/L |
| Yeast extract | 2 g/L |
| FeSO₄ | 0.01 g/L |
| MnSO₄ | 0.01 g/L |
| CaCO₃ | 30 g/L (separately sterilized) |
| NaCl | 15 g/L |

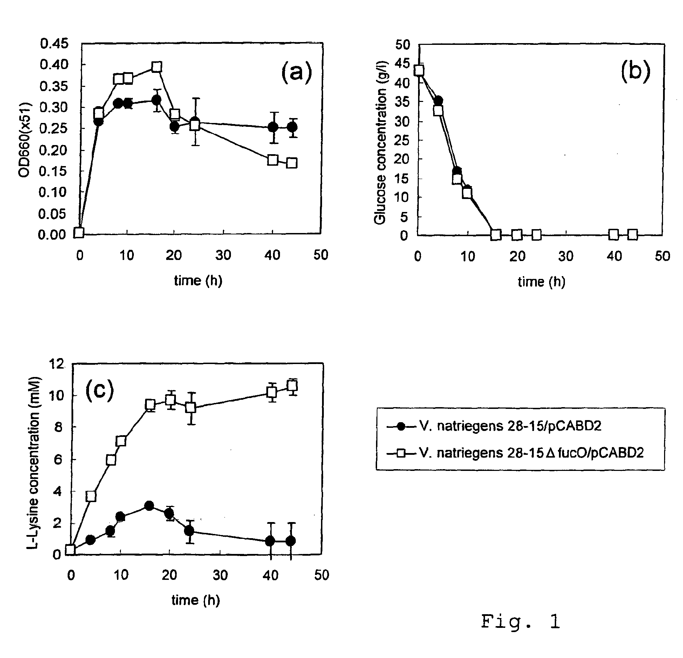

As a result, with *V. natriegens* 28-15 Δfuco/pCABD2, accumulation of 10 mM L-lysine was observed, and decomposition of L-lysine was not observed after glucose was completely consumed (Fig. 1). On the other hand, with the strain in which the *fucO* gene was not disrupted, although accumulation of 3 mM L-lysine was observed at most, decomposition of L-lysine was observed after that. That is, with the *fucO*-deficient strain, L-lysine accumulation higher by more than 3 times was observed, and decomposition of L-lysine was also suppressed, as compared to the strain in which the *fucO* gene was not deleted.

As described above, there was suggested a possibility that the *fucO* gene was involved in decomposition of L-lysine in *V. natriegens.* Therefore, L-lysine decomposition pathway of *V. natriegens* may differ from the pathway through which cadaverine is generated from L-lysine.

### Example 3

Then, with *V. natriegens* 28-15 ΔfucO/pCABD2, L-lysine production was performed under a high salt concentration by using glucose as the carbon source. *V. natriegens* 28-15 ΔfucO/pCABD2 was cultured on the LB-NaCl agar medium (containing 500 mg/L of streptomycin) at 37°C for 8 hours. The obtained cells were scraped together, inoculated into 20 ml of the MS medium (containing 500 mg/L of streptomycin, and 0 to 8% (w/v) NaCl) contained in a Sakaguchi flask (volume: 500 ml) at OD660 of 0.1, and cultured at 37°C with shaking. The strain was cultured in three flasks for each salt concentration. The medium was periodically sampled in a volume of 1 ml, and OD660 as well as glucose concentration and L-lysine concentration in the medium were measured. OD660 was measured for the culture medium diluted with 51 times with a 2% (w/v) NaCl aqueous solution by using a spectrophotometer DU-800 (Beckman Coulter, USA). The glucose concentration and L-lysine concentration in the medium were measured by using Biotech Analyzer AS-300 (Sakura Seiki Co., Ltd., Japan).

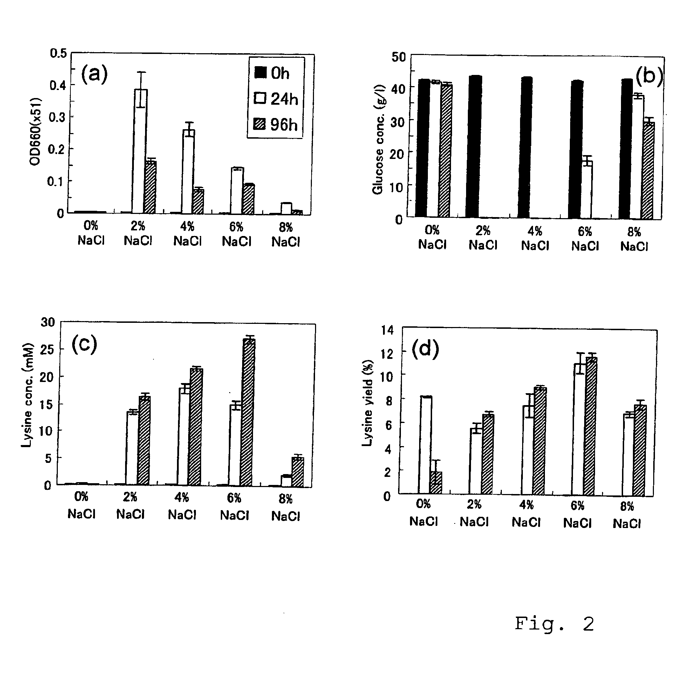

As a result, with the culture condition of adding 6% (w/v) NaCl, accumulation of 27 mM L-lysine at most was observed, and the L-lysine yield based on consumed saccharide at that time was 12 % (w/w) (Fig. 2).

### Example 4

Then, with *V. natriegens* 28-15 ΔfucO/pCABD2, L-lysine production was performed by using glycerol" as the carbon source. Specifically, *V. natriegens* 28-15 Δfuco/pCABD2 was cultured on the LB-NaCl agar medium (containing 500 mg/L of streptomycin) at 37°C for 8 hours. The obtained cells were scraped together, inoculated into 20 ml of the MS medium (containing 500 mg/L of streptomycin, and 2% or 4% (w/v) NaCl) at OD660 of 0.1, and cultured at 37°C with shaking. As the glycerol of the carbon source, glycerol produced by Junsei Chemical Co., Ltd., Japan was used. The strain was cultured in three flasks for each salt concentration. The medium was periodically sampled in a volume of 1 ml, and OD660 as well as glycerol concentration and L-lysine concentration in the medium were measured. OD660 was measured for the culture medium diluted with 51 times with a 2% (w/v) NaCl aqueous solution by using a spectrophotometer DU-800 (Beckman Coulter, USA). The glycerol concentration in the medium was measured by using Biosensor BF-5 (Oji Scientific Instruments Co., Ltd., Japan), and the L-lysine concentration was measured by using Biotech Analyzer AS-300 (Sakura Seiki Co., Ltd., Japan).

As a result, with the culture condition of adding 4% (w/v) NaCl, accumulation of 30 mM L-lysine at most was observed, and the L-lysine yield based on consumed saccharide was 15 % (w/w) (Fig. 3).

### Example 5

In the same manner as that of Example 1 used for obtaining *V. natriegens* 28-15 ΔfucO, except that a *V. natriegens* wild-type strain, ATCC 14048 strain (NBRC 15636, AJ13670), was used instead of the *V. natriegens* 28-15 strain, a *fucO* gene-disrupted strain, VLD01, was obtained.

### Example 6

With *V. natriegens* 28-15 ΔfucO/pCABD2, VLD01/pCABD2, and *V. natriegens* ATCC 14048/pCABD2, L-lysine production was performed by using glycerol as the carbon source in the MS4Y medium, which is the MS culture excessively supplemented with yeast extract. First, each strain was cultured on the LB-NaCl agar medium (containing 500 mg/L of streptomycin) at 37°C for 8 hours. The obtained cells were scraped together, inoculated into 20 ml of the MS4Y medium at OD660 of 0.1, and cultured at 37°C with shaking. As the glycerol of the carbon source, glycerol produced by Junsei Chemical Co., Ltd., Japan was used. Each strain was cultured in two flasks. The medium was periodically sampled in a volume of 0.5 ml, and OD660 as well as glycerol concentration and L-lysine concentration in the medium were measured. OD660 was measured for the culture medium diluted with 50 times with 1 N HC1 aqueous solution by using a spectrophotometer DU-800 (Beckman Coulter, USA). The glycerol concentration in the medium was measured by using Biosensor BF-5 (Oji Scientific Instruments Co., Ltd., Japan), and the L-lysine concentration was measured by using Biotech Analyzer AS-300 (Sakura Seiki Co., Ltd., Japan).

### [MS4Y medium]

### (Final concentration)

| | |
|---|---|
| Glycerol | 40 g/L (separately sterilized) |
| MgSO₄·7H₂O | 1 g/L (separately sterilized) |
| (NH₄)₂S₄O | 24 g/L |
| KH₂PO₄ | 1 g/L |
| Yeast extract | 8 g/L |
| FeSO₄ | 0.01 g/L |
| MnSO₄ | 0.01 g/L |
| CaCO₃ | 30 g/L (separately sterilized) |
| NaCl | 20 g/L (separately sterilized) |

In the medium excessively supplemented with yeast extract, *V. natriegens* 28-15 ΔfucO/pCABD2 accumulated 61 mM L-lysine (Fig. 4). In this case, the L-lysine yield based on consumed glycerol was 28% (w/w). Furthermore, accumulation of 45 mM L-lysine was also observed with VLD01/pCABD2, and the L-lysine yield based on consumed glycerol was 21% (w/w). With *V. natriegens* ATCC 14048/pCABD2, accumulation of L-lysine was not substantially observed.

### Explanation of Sequence Listing

SEQ ID NO: 1: Nucleotide sequence of *fucO* gene of *V. natriegens*
SEQ ID NO: 2: Amino acid sequence encoded by *fucO* gene of *V. natriegens*
SEQ ID NO: 3: Nucleotide sequence of PCR primer for amplifying *sacB* gene
SEQ ID NO: 4: Nucleotide sequence of PCR primer for amplifying *sacB* gene
SEQ ID NO: 5: Nucleotide sequence of PCR primer for amplifying 0.8 kbp sequences on both sides of *fucO* gene
SEQ ID NO: 6: Nucleotide sequence of PCR primer for amplifying 0.8 kbp sequences on both sides of *fucO* gene
SEQ ID NO: 7: Nucleotide sequence of PCR primer for amplifying 0.8 kbp sequences on both sides of *fucO* gene
SEQ ID NO: 8: Nucleotide sequence of PCR primer for amplifying 0.8 kbp sequences on both sides of *fucO* gene
SEQ ID NO: 9: Nucleotide sequence of PCR primer for confirming disruption of *fucO* gene on chromosome
SEQ ID NO: 10: Nucleotide sequence of PCR primer for confirming disruption of *fucO* gene on chromosome
SEQ ID NO: 11: Nucleotide sequence of *AKO* gene of *V. natriegens*
SEQ ID NO: 12: Amino acid sequence encoded by *AKO* gene of *V. natriegens*
SEQ ID NO: 13: Nucleotide sequence of *thrA* gene of *V. natriegens*
SEQ ID NO: 14: Amino acid sequence encoded by *thrA* gene of *V. natriegens*
SEQ ID NO: 15: Nucleotide sequence of *metL* gene of *V. natriegens*
SEQ ID NO: 16: Amino acid sequence encoded by *metL* gene of *V. natriegens*
SEQ ID NO: 17: Nucleotide sequence of *lysC* gene of *V. natriegens*
SEQ ID NO: 18: Amino acid sequence encoded by *lysC* gene of *V. natriegens*
SEQ ID NO: 19: Nucleotide sequence of putative-AK gene of *V. natriegens*
SEQ ID NO: 20: Amino acid sequence encoded by putative-AK gene of *V. natriegens*

### Industrial Applicability

According to the method of the present invention, L-lysine can be efficiently produced by using a *Vibrio* bacterium.

In particular, with a *Vibrio* bacterium deficient in the *fucO* gene, decomposition of L-lysine accumulated with time is suppressed, and thus L-lysine can be very efficiently accumulated.

## Claims

1. A method for producing L-lysine, which comprises culturing a *Vibrio* bacterium having an ability to produce L-lysine in a medium to produce and accumulate L-lysine in the medium or cells of the bacterium, and collecting L-lysine from the medium or cells, wherein the *Vibrio* bacterium has been modified so that an activity of a protein encoded by the *fucO* gene is reduced.

2. The method according to claim 1, wherein the activity of the protein is reduced by introducing a mutation into a coding region of the *fucO* gene and/or an expression control region of the gene.

3. The method according to claim 1 or 2, wherein, the *fucO* gene on a chromosome is disrupted.

4. The method according to any one of claims 1 to 3,
wherein the protein is a protein defined in the following (A) or (B):
(A) a protein having the amino acid sequence shown in SEQ ID NO: 2,
(B) a protein having the amino acid sequence shown in SEQ ID NO: 2 including substitutions, deletions, insertions or additions of one or several amino acid residues, wherein the reduction of the activity in the bacterium improves the ability to produce L-lysine.

5. The method according to any one of claims 1 to 4,
wherein the *fucO* gene is a DNA defined in the following (a) or (b) :
(a) a DNA comprising the nucleotide sequence of SEQ ID NO: 1,
(b) a DNA which hybridizes with a DNA complementary to the nucleotide sequence of SEQ ID NO: 1 under stringent conditions of 1 x SSC, 0.1 % SDS at 60 °C, and codes for a protein, wherein the reduction of the activity in the bacterium improves the ability to produce L-lysine.

6. The method according to any one of claims 1 to 5,
wherein an activity or activities of one or two or more kinds of enzymes selected from the group consisting of dihydrodipicolinate synthase, aspartokinase, dihydrodipicolinate reductase, and diaminopimelate dehydrogenase are enhanced.

7. The method according to any one of claims 1 to 6,
wherein the *Vibrio* bacterium is *Vibrio natriegens.*

8. The method according to any one of claims 1 to 7,
wherein the medium contains glycerol as a carbon source.

9. A *Vibrio* bacterium which has an ability to produce L-lysine, and has been modified so that an activity of a protein encoded by the *fucO* gene is reduced.

10. The *Vibrio* bacterium according to claim 9, which is *Vibrio natriegens.*

## Patentansprüche

1. Verfahren zum Herstellen von L-Lysin, welches das Kultivieren eines *Vibrio*-Bakteriums mit der Fähigkeit zur Produktion von L-Lysin in einem Medium, wobei L-Lysin in dem Medium oder Zellen des Bakteriums hergestellt und angehäuft wird, und das Gewinnen von L-Lysin aus dem Medium oder Zellen umfasst, wobei das *Vibrio*-Bakterium so modifiziert worden ist, dass eine Aktivität eines durch das *fucO*-Gen kodierten Proteins verringert ist.

2. Verfahren nach Anspruch 1, wobei die Aktivität des Proteins durch Einführen einer Mutation in eine kodierende Region des *fucO*-Gens und/oder eine Expressionskontrollregion des Gens verringert ist.

3. Verfahren nach Anspruch 1 oder 2, wobei das *fucO*-Gen auf einem Chromosom unterbrochen ist.

4. Verfahren nach einem der Ansprüche 1 bis 3, wobei das Protein ein Protein gemäß der folgenden Definition (A) oder (B) ist:
(A) ein Protein mit der in SEQ ID NR. 2 gezeigten Aminosäuresequenz,
(B) ein Protein mit der in SEQ ID Nr. 2 gezeigten Aminosäuresequenz, einschließlich Substitutionen, Deletionen, Insertionen oder Additionen von einem oder mehreren Aminosäureresten, wobei die Verringerung der Aktivität in dem Bakterium die Fähigkeit zur Produktion von L-Lysin verbessert.

5. Verfahren nach einem der Ansprüche 1 bis 4, wobei das *fucO*-Gen eine DNA gemäß der folgenden Definition (a) oder (b) ist:
(a) eine DNA, welche die Nucleotidsequenz der SEQ ID NR. 1 umfasst,
(b) eine DNA, die mit einer zu der Nucleotidsequenz der SEQ ID NR. 1 komplementären DNA unter stringenten Bedingungen von 1 x SSC, 0,1% SDS bei 60°C hybridisiert und für ein Protein kodiert, wobei die Verringerung der Aktivität in dem Bakterium die Fähigkeit zur Produktion von L-Lysin verbessert.

6. Verfahren nach einem der Ansprüche 1 bis 5, wobei eine Aktivität oder Aktivitäten von einer oder von zwei oder mehr Arten von Enzymen, die aus der aus Dihydrodipicolinatsynthase, Aspartokinase, Dihydrodipicolinatreductase und Diaminopimelatdehydrogenase bestehenden Gruppe ausgewählt sind, verstärkt ist/sind.

7. Verfahren nach einem der Ansprüche 1 bis 6, wobei das *Vibrio*-Bakterium *Vibrio natriegens* ist.

8. Verfahren nach einem der Ansprüche 1 bis 7, wobei das Medium Glycerin als Kohlenstoffquelle enthält.

9. *Vibrio*-Bakterium, welches die Fähigkeit zur Produktion von L-Lysin hat und so modifiziert worden ist, dass eine Aktivität eines von dem *fucO*-Gen kodierten Proteins verringert ist.

10. *Vibrio*-Bakterium nach Anspruch 9, welches *Vibrio natriegens* ist.

## Revendications

1. Procédé pour produire de la L-lysine, qui comprend la culture d'une bactérie *Vibrio* ayant une aptitude à produire de la L-lysine dans un milieu pour produire et accumuler de la L-lysine dans le milieu ou des cellules de la bactérie, et la collecte de L-lysine à partir du milieu ou des cellules, où la bactérie *Vibrio* a été modifiée de sorte qu'une activité d'une protéine codée par le gène *fucO* est réduite.

2. Procédé selon la revendication 1, où l'activité de la protéine est réduite par introduction d'une mutation dans une région codante du gène *fucO* et/ou une région de contrôle de l'expression du gène.

3. Procédé selon la revendication 1 ou 2, où, le gène *fucO* sur un chromosome est interrompu.

4. Procédé selon l'une quelconque des revendications 1 à 3, où la protéine est une protéine définie dans (A) ou (B) suivant :
(A) une protéine ayant la séquence d'aminoacides montrée dans SEQ ID NO:2,
(B) une protéine ayant la séquence d'aminoacides montrée dans SEQ ID NO:2 incluant des substitutions, des délétions, des insertions ou des additions d'un ou plusieurs résidus d'aminoacides, où la réduction de l'activité dans la bactérie améliore l'aptitude à produire de la L-lysine.

5. Procédé selon l'une quelconque des revendications 1 à 4, où le gène *fucO* est un ADN défini dans (a) ou (b) suivant :
(a) un ADN comprenant la séquence nucléotidique de SEQ ID NO:1,
(b) un ADN qui s'hybride avec un ADN complémentaire de la séquence nucléotidique de SEQ ID NO:1 dans des conditions stringentes de 1 x SCC, 0,1 % SDS à 60°C, et code une protéine, où la réduction de l'activité dans la bactérie améliore l'aptitude à produire de la L-lysine.

6. Procédé selon l'une quelconque des revendications 1 à 5, où une activité ou des activités d'un ou deux ou plusieurs types d'enzymes choisies dans le groupe consistant en dihydrodipicolinate synthase, aspartokinase, dihydrodipicolinate réductase, et diaminopimélate déshydrogénase sont augmentées.

7. Procédé selon l'une quelconque des revendications 1 à 6, où la bactérie *Vibrio* est *Vibrio natriegens.*

8. Procédé selon l'une quelconque des revendications 1 à 7, où le milieu contient du glycérol comme source de carbone.

9. Bactérie *Vibrio* qui a une aptitude à produire de la L-lysine, et a été modifiée de sorte qu'une activité d'une protéine codée par le gène *fucO* est réduite.

10. Bactérie *Vibrio* selon la revendication 9, qui est *Vibrio natriegens.*
